(19) Europäisches Patentamt
European Patent Office
Office européen des brevets

(11) **EP 4 711 706 A1**

(12) **EUROPEAN PATENT APPLICATION**

(43) Date of publication:
**18.03.2026 Bulletin 2026/12**

(21) Application number: **24200929.8**

(22) Date of filing: **17.09.2024**

(51) International Patent Classification (IPC):
**G01B 9/02** $^{(2022.01)}$ **A61B 5/00** $^{(2006.01)}$
**G01B 9/02091** $^{(2022.01)}$

(52) Cooperative Patent Classification (CPC):
**G01B 9/02091; A61B 5/0066; G01B 9/02045;
G01B 9/02087**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Carl Zeiss Meditec AG
07745 Jena (DE)**

(72) Inventors:
• **MacDougall, Daniel
  Halifax, B3H0A8 (CA)**
• **Adamson, Robert
  Halifax, B3H0A8 (CA)**
• **Hubley, Drew
  Halifax, B3H 0A8 (CA)**

(74) Representative: **Carl Zeiss AG - Patentabteilung
Carl-Zeiss-Straße 22
73447 Oberkochen (DE)**

(54) **SYSTEMS AND METHODS FOR ESTIMATING THE VELOCITY OF RIGID STRUCTURES IN THE PRESENCE OF SPECKLE AND MOTION ARTEFACTS**

(57) Systems and methods are provided for performing Doppler (phase-sensitive) optical coherence tomographic vibrometry measurements involving the vibrometric response of a rigid structure to an acoustic stimulus, such that the impact of speckle noise on a calculated vibrometric measure is reduced. Detected signals are processed to determine a depth range associated with the rigid structure, and to provide sampled time-dependent phase data encoded with the vibratory response of the rigid structure during application of the acoustic stimulus. The sampled time-dependent phase profile is processed to generate a vibrometric measure characterizing the rigid structure, based on non-uniformly-weighted contributions from one or more subregions within the depth region associated with the rigid structure (and optionally one or more time windows). The contribution from each subregion may be dependent on a depth-domain intensity measure associated with the subregion, where each subregion may be sufficiently small to encompass speckle-induced spatially-dependent changes.

300

Employ Doppler OCT vibrometry system to interrogate rigid structure along a depth axis — 310

Obtain OCT dataset including Doppler OCT vibrometry dataset obtained during application of acoustic stimulus — 320

Process OCT dataset to identify depth region associated with rigid structure — 330

Process Doppler OCT vibrometry dataset to generate estimated vibration based on contributions from subregions within the depth region such that contribution from each subregion to the estimated vibration is dependent on depth domain intensity measure associated with subregion — 340

**FIG. 3**

**Description**

**TECHNICAL FIELD**

The present disclosure relates to the use of optical coherence tomography for the Doppler (phase-sensitive) detection of acoustic vibrations in the presence of noise. In particular, the present disclosure relates to Doppler (phase-sensitive) optical coherence tomographic vibrometry.

**SUMMARY**

Systems and methods are provided for performing Doppler (phase-sensitive) optical coherence tomographic (OCT) vibrometric measurements of the vibrometric response of a rigid structure to a stimulus, such that the impact of noise on an estimated vibrometric measure for the rigid structure is reduced. Detected signals are processed to determine a depth range associated with the rigid structure, and to provide time-dependent phase data encoded with the vibratory response of the rigid structure during application of the stimulus. The time-dependent phase is processed to generate an estimate of a vibrometric measure characterizing the motion of a rigid structure, based on non-uniformly weighted contributions from one or more subregions within the depth region associated with the rigid structure and optionally one or more time windows, such that the contribution from each subregion and/or time window is dependent on an intensity measure associated with the subregion or time window, where each subregion is sufficiently small to encompass speckle-induced spatially-dependent variation in OCT A-line intensity and each time window is shorter than the time over which the speckle pattern decorrelates due to relative motion between the OCT system and the rigid structure.

According to an aspect of the present disclosure, a method of assessing a vibrometric response of a rigid structure via optical coherence tomography Doppler vibrometry includes employing an optical coherence tomography Doppler vibrometry system to interrogate the rigid structure along a depth axis, and obtaining an optical coherence tomography dataset, the optical coherence tomography dataset including an optical coherence tomography Doppler vibrometry dataset obtained while applying a stimulus, processing the optical coherence tomography dataset to identify a depth region, residing along the depth axis, that is associated with the rigid structure; and processing the optical coherence tomography Doppler vibrometry dataset to generate a vibrometric measure characterizing a vibrometric response of the rigid structure to the stimulus, wherein the vibrometric measure is generated based on non-uniformly weighted contributions from one or more subregions within the depth region associated with the rigid structure.

In some embodiments, the non-uniformly weighted contributions are updated during a plurality of time intervals to provide a plurality of updated values of the non-uniformly weighted contributions, wherein each time interval is shorter in duration than a decoherence time of a speckle pattern, and wherein the updated values are employed to generate the vibrometric measure.

In some embodiments, the non-uniformly weighted contribution from each subregion is dependent on a depth-domain A-line intensity measure associated with the subregion.

In some embodiments, each subregion is sufficiently small to encompass speckle-induced spatially-dependent changes in depth-domain A-line intensity within the depth region.

In some embodiments, each subregion corresponds to a depth-domain voxel.

In some embodiments, the vibrometric measure is generated based on a contribution from a single subregion having a highest depth-domain A-line intensity measure among a set of subregions residing within the depth region.

In some embodiments, the vibrometric measure may be generated by processing the optical coherence tomography Doppler vibrometry dataset to obtain a plurality of A-lines, and generating, from the plurality of A-lines, and for each subregion of a plurality of subregions residing with the depth region, an optical phase time series characterizing a temporal evolution of an optical phase during application of the stimulus, thereby obtaining a set of optical phase time series, each optical phase time series corresponding to a different subregion, and generating, from the set of optical phase time series, an aggregate optical phase time series, such that a given optical phase data point in the aggregate optical phase time series, corresponding to a given time point, is determined as a statistical measure characterizing a distribution of optical phase values among the plurality of subregions at the given time point, and processing the aggregate optical phase time series to determine the vibrometric measure.

In some embodiments, the optical coherence tomography Doppler vibrometry dataset is processed, prior to determining the vibrometric measure, to remove or reduce low-frequency motion noise.

In some embodiments, the stimulus is an acoustic stimulus.

According to an aspect of the present disclosure, a system for assessing a vibrometric response of a rigid structure via optical coherence tomography Doppler vibrometry, the system including an optical coherence tomography Doppler vibrometry system capable for applying a stimulus to the rigid structure, and control and processing circuitry including at least one processor and associated memory, the memory including instructions executable by the at least one processor for performing operations including employing the optical coherence tomography Doppler vibrometry system to interrogate the rigid structure along a depth axis, and obtaining an optical coherence tomography dataset, the optical coherence tomography dataset including an optical coherence tomography Doppler vibrometry dataset obtained while applying the stimulus; processing the optical coherence tomography dataset to identify a depth region, residing along the depth axis, that is associated with the rigid structure, and processing the optical coherence tomography Doppler vibrometry dataset to generate a vibrometric measure characterizing a vibrometric response of the rigid structure to the stimulus, wherein the vibrometric measure is generated based on non-uniformly weighted contributions from one or more subregions within the depth region associated with the rigid structure.

In some embodiments, the control and processing circuitry is configured such that the non-uniformly weighted contributions are updated during a plurality of time intervals to provide a plurality of updated values of the non-uniformly weighted contributions, wherein each time interval is shorter in duration than a decoherence time of a speckle pattern, and wherein the updated values are employed to generate the vibrometric measure.

In some embodiments, the control and processing circuitry is configured such that the non-uniformly weighted contribution from each subregion is dependent on a depth-domain A-line intensity measure associated with the subregion.

In some embodiments, the control and processing circuitry is configured such that each subregion is sufficiently small to encompass speckle-induced spatially-dependent changes in depth-domain A-line intensity within the depth region.

In some embodiments, the control and processing circuitry is configured such that the vibrometric measure is generated based on a contribution from a single subregion having a highest depth-domain A-line intensity measure among a set of subregions residing within the depth region.

In some embodiments, the control and processing circuitry is configured such that the vibrometric measure is generated by: processing the optical coherence tomography Doppler vibrometry dataset to obtain a plurality of A-lines, and generating, from the plurality of A-lines, and for each subregion of a plurality of subregions residing with the depth region, an optical phase time series characterizing a temporal evolution of an optical phase during application of the stimulus, thereby obtaining a set of optical phase time series, each optical phase time series corresponding to a different subregion; and generating, from the set of optical phase time series, an aggregate optical phase time series, such that a given optical phase data point in the aggregate optical phase time series, corresponding to a given time point, is determined as a statistical measure characterizing a distribution of optical phase values among the plurality of subregions at the given time point; and processing the aggregate optical phase time series to determine the vibrometric measure.

A further understanding of the functional and advantageous aspects of the disclosure can be realized by reference to the following detailed description and drawings.

## BRIEF DESCRIPTION OF THE FIGURES

The accompanying drawings are incorporated herein and form a part of the specification.

FIG. 1 illustrates a system for performing Doppler OCT vibrometry, according to some embodiments.

FIG. 2 illustrates an optical coherence tomography image with associated speckle noise, according to some embodiments.

FIG. 3 illustrates a method for performing Doppler OCT vibrometric measurements of acoustic vibrations with reduced susceptibility to speckle noise, according to some embodiments.

FIG. 4 illustrates the spatial and temporal optical phase data, extracted from a set of interferograms acquired during the application of an acoustic stimulus, with the voxels being associated with the rigid structure, according to some embodiments.

FIG. 5 illustrates an algorithm flowchart for calculating a vibrometric measure from a Doppler OCT vibrometry dataset using a single pixel fit algorithm, according to some embodiments.

FIG. 6 illustrates an algorithm flowchart for calculating a vibrometric measure from a Doppler OCT vibrometry dataset using a fitted brightness weighted algorithm, according to some embodiments.

FIG. 7 illustrates an algorithm flowchart for calculating a vibrometric measure from a Doppler OCT vibrometry dataset using a phase median algorithm, according to some embodiments.

FIGS. 8A and 8B illustrate results of a simulation comparing the estimated vibration amplitude when the true amplitude is zero for the methods illustrated in FIGS. 5-7, according to some embodiments.

FIG. 9 illustrates control and processing circuitry for controlling a Doppler OCT vibrometry system, according to some embodiments.

In the drawings, like reference numbers generally indicate identical or similar elements. Additionally, generally, the left-most digit(s) of a reference number identifies the drawing in which the reference number first appears.

**DETAILED DESCRIPTION**

In Doppler (phase-sensitive) optical coherence tomographic vibrometry, the motion of a structure located at a spatial location produces a phase variation between successive amplitude scan lines (A-lines). In Fourier Domain OCT systems, A-lines are produced by taking the inverse Fourier transform (FT) of the sampled spectral interferogram (in swept-source OCT) or the sampled spectrum (in spectral domain OCT). In the absence of noise, changes to the measured optical phase at each vibrating pixel is proportional to the displacement of the structure at that pixel. In real systems, however, there are sources of noise that contaminate the optical phase signal. The phase noise present in OCT systems is non-stationary both space and time. When noise that is additive, Gaussian, independent and identically distributed noise on the spectral interferogram or sampled spectrum, the variance of the phase noise is inversely proportional to the intensity of the A-line at the pixel location. OCT A-lines systems are subject to modulation by speckle caused by the random constructive and destructive interference of multiple reflectors present within the coherence length and area of the OCT beam. Because of speckle, at any moment in time, some pixels in the images will have very low A-line intensity and so very high phase noise variance.

When an estimate is made of the vibration amplitude of a rigid structure based on the measured phase of the OCT A-lines, the inclusion of these low SNR pixels can result in a low accuracy estimate of the vibration amplitude. When the structure moves relative to OCT imaging system, which pixels exhibit constructive and destructive interference due to speckle change, so that pixels that had low SNR at one time could potentially have high SNR at a later time. Because of this effect, the degree to which pixels contribute noise to a composite estimate of vibration amplitude evolves over time. Estimates of the vibration of rigid structures made from OCT A-line data suffer from inaccuracy because of these effects.

As described herein, speckle noise is a type of noise that can contaminate OCT signals and lead to poor estimates of vibrometric measures derived from Doppler OCT. Speckle noise results from the coherent nature of the light employed for optical coherence tomography detection. Speckle noise manifests as granular patterns in the transformed A-line data obtained from an OCT system, resulting from random interference of light scattered by tissue. Speckle noise does not only degrade image quality, but it also leads to the formation of speckle holes, which are pockets of low intensity within the image. Speckle holes result in regions with poor signal-to-noise ratio (SNR), compromising the ability to accurately detect and interpret fine structural details and leading noise in the optical phase associated with Doppler OCT.

When processing Doppler OCT vibrometry data to generate vibrometric measures characterizing a rigid structure, the calculation of the vibrometric measure may be improved by averaging A-line data among a set of voxels spanning the rigid structure, since each of these voxels is expected to vibrate in unison due to the rigidity of the rigid structure. However, it was found that the signal-to-noise ratio of the aggregate measure was still compromised by the presence of speckle holes within the A-line data across the spatial extent of the rigid structure.

The present inventors realized that when employing A-line data to generate a vibrometric data based on voxels spanning a rigid structure, an improvement in signal-to-noise ratio (SNR) of the vibrometric measure could be obtained by non-uniformly weighting the contribution of the voxel to the vibrometric measure based on the A-line intensity (in the depth domain) of the voxel (or alternatively, of a group of voxels).

Accordingly, in some embodiments, Doppler (phase-sensitive) optical coherence tomographic vibrometric measurements are obtained that characterize the vibrometric response of a rigid structure to an acoustic stimulus (e.g. acoustic or mechanical) and are processed in such a manner that the impact of speckle noise on a calculated vibrometric measure is suppressed or reduced. As described in detail below, detected OCT signals are processed to determine a depth range associated with the rigid structure, and to provide sampled time-dependent phase data encoded with the vibratory response of the rigid structure during application of the stimulus. The sampled time-dependent phase profile is processed to generate a vibrometric measure characterizing the rigid structure, based on non-uniformly weighted contributions from one or more subregions within the depth region associated with the rigid structure (and optionally one or more time windows), such that the contribution from each subregion is dependent on a depth-domain intensity measure associated with the subregion, where each subregion may be sufficiently small to encompass speckle-induced spatially-dependent changes.

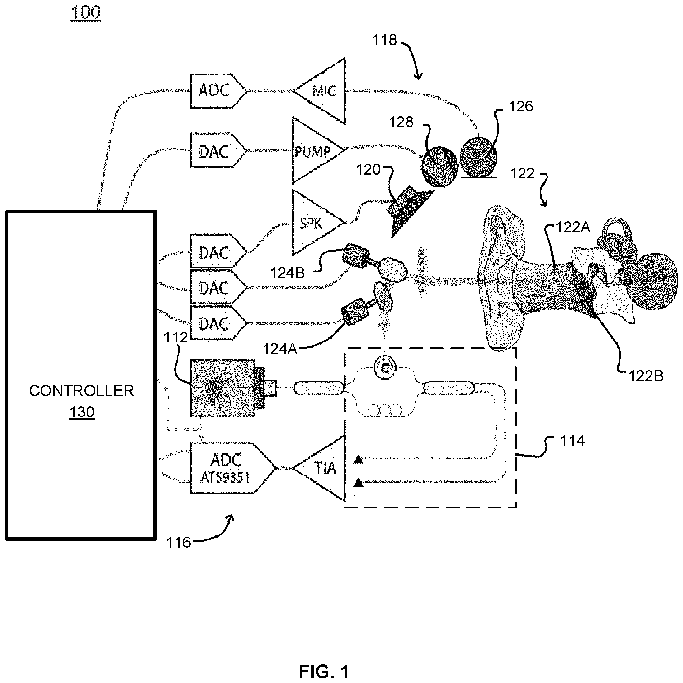

FIG. 1 illustrates a Doppler OCT optical coherence tomography vibrometry system 100, according to some embodiments. This non-limiting example system includes an akinetic swept-source laser 112, an interferometer 114, high speed digitization electronics 116, and a sound delivery system 118 for delivering a sound stimulus to an ear 122 via a speaker 120. Laser light from the interferometer 114 is directed into the ear 122 by scanning mirrors 124A and 124B, and scattered light is collected and directed back to the interferometer 114, this forming the sample arm of the interferometer 114.

The sound stimulus provided by speaker 120 and is synchronized to a sweep clock signal of laser 112 in such a way that the acoustic phase of the stimulus is advanced in coordination with each sweep of laser 112. The sweep clock signal is a clock signal generated by laser 112 or used to drive laser 112 such that an edge of the sweep clock signal occurs in a fixed timing relationship to the beginning of the frequency sweep of laser 112. Synchronization of the sweep clock signal and sound stimulus ensures that the measured optical phase in the resulting image of ear 122 will advance at the same rate as the phase of the acoustic stimulus resulting in a stable phase relationship between the acoustic stimulus and the measured optical phase at each voxel in the image at each stimulus frequency.

The microphone 126 records the pressure in ear canal 122A in response to the acoustic stimulus generated by speaker 120. A pump 128 may be provided to maintain a desired static pressure in ear canal 122A. While many embodiments herein describe the use of an acoustic stimulus, in other embodiments the stimulus may be a mechanical stimulus.

The laser 112, speaker 120, scanning mirrors 124A and 124B, microphone 126, and pump 128 may all be connected to a controller 130. Controller 130 processes signals from interferometer 114 to produce an interferogram representing one or more structures and/or encoding vibrometric features of one or more structures within the ear canal 122A and/or eardrum 122B (nonlimiting examples of such structures include rigid bony structures such as the ossicles (malleus, incus and stapes)). Scanning mirrors 124A and 124B may scan the beam from laser 112 laterally across the field of view.

While FIG. 1 shows a mirror-based scanning subsystem, in other example embodiments, one or more of the mirrors may be replaced by other scanning elements. Non-limiting examples of non-mirror based scanning elements include acousto-optic deflectors, electro-optic deflectors, liquid crystal spatial light modulators, and photorefractive scanners.

In some embodiments, a Doppler OCT vibrometric subsystem may be integrated with a handheld probe that includes the scanning mirrors 124A and 124B and is configured for use with a disposable speculum, similar to an otoscope, similar to an otoscope, that is inserted into ear canal 122A. One or more tubes may be integrated into the handheld probe to carry the acoustic stimulus, connect to microphone 126 and/or deliver a static pressure to ear canal 122A. When a seal is required, a foam or silicone sheath can be placed around the speculum in order to seal ear canal 122A when the speculum is inserted. Controls (e.g. buttons and a selection wheel) may be incorporated into the handheld probe or may be provided in a remote configuration, for example to allow the clinician to select different imaging and measurement modes and to switch between B-mode OCT measurement and Doppler vibrometric measurements, or other types of diagnostic measurements.

In other embodiments the imaging head may be integrated into a surgical microscope. In yet other embodiments, the imaging head may be fixed to a frame used to stabilize the patient's head. The apparatus as described herein may be applied, for example, by otologists and audiologists. The apparatus may, for example, be applied in clinical settings and intrasurgical applications. The handheld probe may include an integrated imaging camera that facilitates combined optical imaging and OCT imaging/measurement.

In some embodiments, Doppler OCT vibrometry may be performed using a swept-source laser 112, while in other embodiments, Doppler OCT vibrometry may be performed using a broadband, low-coherence light source in a spectral-domain OCT vibrometry configuration. In yet other embodiments, Doppler OCT vibrometry may be performed using a broadband, low-coherence light source and time-domain Doppler OCT vibrometry system.

It will be understood that the systems and methods described herein need not be implemented according to the hardware configurations described herein and shown in FIG. 1. In some embodiments, other computer hardware may be employed as processing circuitry to perform the collection, synchronization, and analysis, in which processing circuitry, including at least one processor and memory, is operably connected to the Doppler optical coherence tomography subsystem to receive the A-line trigger therefrom, and is configured such that the at least one processor is configured to execute instructions stored in the memory for performing the methods disclosed herein.

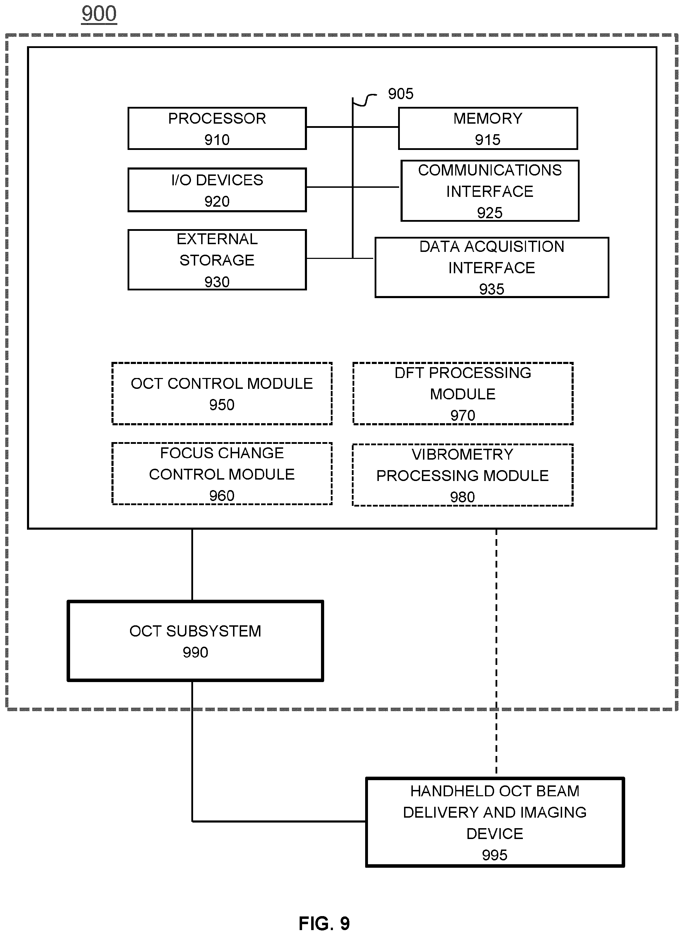

In some embodiments, one or more GPUs are employed to perform the OCT data acquisition and analysis, as further described in FIG. 9. The processing may be performed on a CPU. In yet another embodiment, the processing may be performed on a FPGA in communication with a computer. In some embodiments, data collection is performed by an FPGA or other programmable processor with an embedded analog-to-digital converter that is synchronized to the laser sweep trigger, wherein raw sampled data is digitally transferred to a host computer that performs the processing. In some embodiments, data acquisition is performed by an FPGA or other programmable processor with an embedded analog-to-digital converter that is synchronized to the laser sweep trigger, wherein the sampled data is collected and partly or completely processed locally on embedded programmable hardware before transmission and completion of processing on a host computer.

As described above, FIG. 1 provides a system 100 for performing Doppler OCT vibrometric measurements. It is noted that this system 100 provides but one example system for performing Doppler OCT measurements, and that other systems, such as a spectral domain Doppler OCT system of any configuration could be used as platform to perform the phase measurements and vibrometric measure estimation described herein. The example system shown in FIG. 1 is described in International Patent Application No. PCT/CA2018/051255, titled "SYSTEMS AND METHODS FOR MIDDLE EAR IMMITANCE TESTING" and filed on October 4, 2018, which is incorporated herein by reference in its entirety. Other non-limiting examples of swept-source Doppler OCT systems are described in International Patent Application No. PCT/CA2016/051199, titled "SYSTEMS AND METHODS FOR SWEPT SOURCE OPTICAL COHERENCE TOMOGRAPHY VIBROGRAPHY" and filed on October 14, 2016, which is incorporated herein by reference in its entirety, and in International Patent Application No. PCT/CA2018/050200 (incorporated by reference as noted above).

The example OCT vibrometry system shown in FIG. 1 may employ a swept-source akinetic laser 112, such as the SLE-101E akinetic laser from Insight Photonics Solutions, which provides a sweep frequency of 100 kHz and a fiber-based interferometer 114. The swept source laser 112 provides a sample clock and a sweep trigger, where the sweep trigger signals the beginning of each new laser sweep (each A-line event corresponding to an acquired interferogram). The sweep trigger may be derived from the sample clock. The sample clock and sweep trigger supply timing signals to other clocked and temporally synchronized components of the OCT vibrometry system. In some embodiments, the akinetic laser thus acts as the timing master to other system components, which themselves act as timing-slaves.

The sample clock and the sweep trigger are provided to a data acquisition card (e.g., ATS9351, 12-bit, 500 MHz PCIE digitizer, Alazar Technologies) to synchronize laser sweeping and data acquisition. The sweep trigger is also used to clock internal functions of the controller, which may include a field-programmable-gate-array that forms a component of controller 130 (e.g., MachXO2-7000HE, Lattice Semiconductor) that, through digital-to-analog converters (DAC), respectively control the scanning optics 124A and 124B and the acoustic stimulus source 120 that generates an acoustic stimulus signal for excitation of the object of interest, such as structures within the middle ear.

A digital synthesis approach may be employed to generate the scanning signals and acoustic signals. As shown in FIG. 1, the controller 130 is employed to send control signals to components of the system, but the synchronization of the optical beam scanning and the phase of the acoustic stimulus is determined by the sweep trigger of the laser 112.

The use of the single clock generated by the akinetic laser to synchronize the scanning mirrors 120 and the acoustic stimulus source 120 provides the advantage of synchronously controlling the phase of the acoustic stimulus during optical scanning. In the present example embodiment shown in FIG. 1, a synchronized acoustic stimulus signal is generated by the FPGA (controller 130) by incrementing a counter on the rising edge of each clock cycle (i.e., on each laser sweep). This counter acts as a phase accumulator and is used to step though a lookup table containing the values of a sinusoid of a particular acoustic frequency. On each clock edge, the FPGA updates the value of a digital-to-analog converter (e.g., DAC900E, Texas Instruments) that drives an audio amplifier and speaker 120 used to excite vibrations in the sample.

Two synchronized lateral digital scanning signals (one for horizontal scanning, and one for vertical scanning) are also generated by the FPGA by incrementing a counter on the rising edge of each laser sweep clock cycle (i.e. in order to count laser sweeps). Once the desired number of laser sweeps for a particular scan configuration (image line) have completed, the scan mirror position is incremented by a chosen step size and the laser sweep counting starts over. This process is repeated once for every line in the image. Once the desired number of steps have been made (i.e. the number of lines in the image), the signal is reset to a chosen initial value and the lateral scan procedure starts over. The FPGA updates the values of two digital-to-analog converters (e.g., DAC900E, Texas Instruments) that respectively trigger mirror drivers (shown as cylinders in FIG. 1) to drive the horizontal and vertical scanning optics / mirrors (shown as polygons in FIG. 1, mounted to their respective driver). In one example embodiment, there are two mirror drivers, each of which drives a mirror.

In some embodiments, the acoustic excitation remains phase-locked to the laser sweeps via the sweep trigger, and the accumulation of acoustic phase during and between consecutive image frames can be controlled and predicted deterministically. Without such phase-locking, even relatively slow-phase drifts between the acoustic stimulus and the laser sweeping would tend to average away the vibrational information. It is the synchronization of the timing components to the akinetic laser clock that allows this simple form of indexing to track acoustic phase changes and makes the processing steps well suited to parallel processing on graphical processing units (GPUs) using, for instance, the Compute Unified Device Architecture (CUDA) framework.

The example system shown in FIG. 1, and the example methods of signal processing described in detail below, are well suited when using akinetic lasers because of the characteristic way that linear sweeping over a broad bandwidth is achieved: through controlled mode-hopping in the akinetic swept-source architecture. The laser executes linear-in-frequency sweeps over a limited frequency range. When the end of that range is reached the laser undergoes a mode-hopping event. During this event the interferogram data is invalid and must be removed prior to line reconstruction. Fortunately, the mode hops are repeatable and deterministic across sweeps, such that, for a given laser calibration and set of sweep settings, the invalid data can be identified. However, the presence of these invalid points and their dependence on calibration and sweep parameters means that a system synchronized to the laser sampling clock (or the "k-clock" in the language conventionally used for swept source lasers) results in a phase error that depends on the sweep settings and calibration. In an example embodiment, synchronizing to the start of the sweep with an external FPGA avoids this phase error.

It will be understood that the system 100 shown in FIG. 1 is not intended to be limiting, and that other Doppler optical coherence tomography imaging system configurations may be employed in the alternative. For example, in some example embodiments, systems and methods described herein can be adapted to spectral domain optical coherence tomography (SD-OCT) vibrometry. SD-OCT systems direct the collected and interfered light onto a spectrometer and the intensity at discrete optical wavelengths is recorded by, for instance, a CMOS detector array, charge-coupled device (CCD) or photodiode array. In SD-OCT systems using this approach, the detector array signal is appropriately resampled to obtain measurements that are linearly spaced in optical frequency, and then Fourier transformed forming an A-line signal. The phase of consecutive A-lines is multiplied by in-phase and quadrature sinusoids and averaged. The embodiments described herein are in the context of swept-source OCT vibrometry may be adapted to SD-OCT systems, for example, by employing the A-line trigger to trigger the acquisition of a spectrum from the detector array, in the place of the sweep trigger, such that the scanning subsystem and the acoustic stimulus subsystem are synchronized with the A-line trigger. In some embodiments, Doppler OCT vibrometry may be performed using a broadband, low-coherence light source and time-domain Doppler OCT vibrometry system.

As illustrated in FIG 1, system 100 and other Doppler OCT vibrometry systems, may be employed for ontological vibrometric measurements. When an acoustic tone is presented to the ear, the middle ear structures (the eardrum and ossicles) are set into periodic motion at the acoustic frequency as they conduct sound energy to the inner ear. When the vibrating structures are interrogated via Doppler (phase-sensitive) OCT vibrometry, phase variations in the interferometric signal appear at the acoustic frequency as well. While the audible range of acoustic frequencies is typically cited as 20 Hz - 20kHz, the range of diagnostically useful frequencies is limited to 100 Hz to 10 kHz.

The system 100 in FIG. 1 may be employed to perform a single vibrometric measurement at a selected lateral scanning location, or, for example, to collect vibrometric image data among a plurality of locations. In some embodiments, multi-frame averaging is employed to interleave B-mode imaging with the generation of vibrometric measures, based on the use of the sweep trigger to control synchronization of the phase evolution of the acoustic stimulus among multiple image frames.

Vibrometry data is obtained from a Doppler OCT vibrometry system by processing multiple interferograms for each A-line and extracting the amplitude and phase of a vibrometric measure describing the vibration of the structure in response to the acoustic stimulus, from the interferograms. A discrete Fourier transform (DFT) is obtained for each sampled interferogram (in swept-source OCT) or sampled spectrum (in spectral domain OCT), yielding, for each pixel associated with a given structure, an optical phase signal characterized by a vibrational response driven by the acoustic stimulus. In the case where the acoustic stimulus is sinusoidal and the system mechanics are linear (as is the case in the middle ear), the vibrational response will also be sinusoidal, will occur at the same frequency as the stimulus and can be fully characterized by a complex dynamic displacement or, equivalently, a displacement magnitude and phase.

In real systems, error sources add noise to the sinusoidal response associated with the vibration of the structure that is induced by the acoustic stimulus. For example, a first type of noise that can contaminate the signal is random broadband noise due to shot noise, residual intensity noise and other random sources that overlaps the measurement passband. Such noise sources usually are additive to the interferogram, with an independent and identically distributed Gaussian distribution. Given adequate time, such random noise sources can be averaged down to an acceptably low level in order to recover the vibratory response. Another type of noise is motion noise that is generated by motion of the subject relative to the OCT optics (unless the subject is a cadaver and is absent of motion). Such motion noise can be caused, for example, by breathing and heartbeat, which can produce very large amplitude and low frequency phase noise that dwarfs the vibratory response of the system during the measurement of phase-sensitive OCT signals. As noted above, in the case of middle ear imaging, the head moves a significant fraction of a millimeter every time the heart beats, but the vibration of the middle ear structures in response to sound may be less than a nanometer - hundreds of thousands of times smaller.

Motion noise, which is non-sinusoidal, cannot be aligned with the sampling window in such a way as to be isolated in a small number of DFT bins due to a "spectral splatter" effect. Furthermore, conventional windowing (i.e. multiplication by a Hanning window) along with averaging is ineffective at removing such a large signal, requiring extremely long DFT lengths (or long temporal averaging of many short duration DFTs). Temporal averaging for more than a few 10s of seconds is impractical in a clinical scenario since patients are generally not able to keep still long enough to reach the desired sensitivity.

Motion noise suppression can be performed according to various known methods that take advantage of the low-frequency nature of motion noise, relative to the acoustic frequency variations of interest, in order to achieve noise suppression, such as, for example, methods disclosed in International Patent Application No. PCT/CA2018/050200, titled "SYSTEMS AND METHODS FOR PERFORMING PHASE-SENSITIVE ACOUSTIC VIBRATIONS USING OPTICAL COHERENCE TOMOGRAPHY" and filed on February 22, 2018, which is incorporated herein by reference in its entirety. For example, heartbeat noise has little spectral content above 100 Hz, whereas clinically relevant acoustic responses typically occur at frequencies above 250 Hz. Motion noise suppression may be achieved by combining different portions of a detected vibrometric response to separate the contribution of the component associated with the acoustic stimulus from the component associated with motion noise. This allows the signal to be processed to remove the noise component and obtain an estimate of the vibrometric response in the absence of motion noise. Alternative methods of motion noise reduction, described in further detail below, include approaches such as the linear fit described below and the use of a high-pass filter.

In a Fourier-domain OCT vibrometry system, a sequence of spectral interferograms $H(k, t)$ are collected with each spectral interferogram consisting of interference amplitudes where $k = 2\pi/\lambda$ denotes the wave number corresponding to optical wavelength $\lambda$ and $t$ denotes the time at which the interferogram was collected. If a sample contains a single reflector at a relative distance to the reference arm of $\Delta z$ and the reflector is vibrating with a displacement $\delta z(t)$ such that $\delta z(t) \ll \lambda_0$ where $\lambda_0$ is the center wavelength of the OCT light source, then the interferogram signal $H(k, t)$ is given by

$$H(k,t) = \frac{\rho}{b} S(k) \sqrt{R_R R_S} \cos(2kn\Delta z + 2k_0 n\delta z(t)) + n_k(k,t), \quad (1)$$

where $\rho$ is the spectral responsivity of the detector, $b = 2$ for a balanced detector system and $b = 1$ for an unbalanced, single-sided detector, $R_R$ and $R_S$ are the reference arm and sample arm reflectivity, $S(k)$ is the power spectrum of the light source, $n$ is the index of refraction of the sample material, and $n_k(k, t)$ is additive noise which is assumed to be independent and identically distributed Gaussian noise.

An inverse Fourier transform may be applied to $H(k, t)$ along the $k$ dimension to recover a sequence of complex A-lines $h(z, t)$ where z denotes spatial depth.

For the situation described above with a single reflector in the sample arm,

$$h(z,t) = \frac{\rho s(0)\sqrt{R_R R_S}}{2b} e^{j2k_0 n\delta z(t)} + n_z(z,t), \quad (2)$$

where s(0) is the time-averaged signal power, and $n_z(z, t)$ is the complex additive noise in the z-domain obtained by inverse Fourier transforming $n_k(k, t)$. From the properties of the Fourier transform, if $n_k$ is an independent, identically distributed Gaussian real random variable with respect to z, then the real and imaginary parts of $n_z$ will be independent, identically distributed Gaussian random variables with respect to z.

The time-domain optical phase $\phi(z, t) \angle h(z, t)$ includes a contribution from the vibratory response of the structure $2k_0 n\delta z(t)$ and a contribution from the noise $n_z(z, t)$. The phase angle of the noise signal is denoted as

$$\phi_n(z,t) = \angle n_z(z,t) = \arctan \frac{Im\{n(z,t)\}}{Re\{n(z,t)\}}.$$

The instantaneous optical phase (including the noise $n_z(z, t)$) is then

$$\phi(z,t) = \angle h(z,t) = \arctan \frac{|n_z(z,t)| \sin[\phi_n(z,t) - 2k_0\delta z(t)]}{\left|\frac{\rho s(0)\sqrt{R_R R_S}}{2b}\right| + |n_z(z)| \cos[\phi_n(z) - 2k_0\delta z(t)]} + 2k_0\delta z(t)$$

$$\approx \frac{|n_z(z,t)| \sin[\phi_n(z) - 2k_0 n\delta z(t)]}{\left|\frac{\rho s(0)\sqrt{R_R R_S}}{2b}\right|} + 2k_0\delta z(t)$$

$$\approx \phi_{noise}(z,t) + \phi_{vib}(z,t) \quad (3)$$

where $\phi_{noise}$ (the phase noise) and $\phi_{vib}$ (the vibratory phase) are components of the instantaneous optical phase attributable to the noise and the vibratory response respectively and $k_0 = \frac{2\pi}{\lambda_0}$. The approximation applies in the common situation where the SNR is high, i.e. where $\left|\frac{\rho s(0)\sqrt{R_R R_S}}{2b}\right| \gg |n_z(z,t)|$.

The variance of phase noise $\phi_{noise}(z, t)$ is given by $\sigma_t^2 = E[\phi_{noise}^2(z,t)]$ where $E[*]$ denotes an expectation value:

$$\sigma_t^2 = \frac{E\left[|n_z^2(z,t)| \sin^2[\phi_n(z,t) - \phi_i(z,t)]\right]}{\left|\frac{\rho s(0)\sqrt{R_R R_S}}{2b}\right|^2} = \frac{E[|n_z^2(z,t)|]}{2\left|\frac{\rho s(0)\sqrt{R_R R_S}}{2b}\right|^2} = \frac{1}{2SNR_z}, \qquad (4)$$

where $SNR_z = \dfrac{\left|\frac{\rho s(0)\sqrt{R_R R_S}}{2b}\right|^2}{E[|n_z^2(z,t)|]}$ is the ratio of the A-line signal power to the A-line noise power at the reflector location z.

Thus, the variance of the phase noise is inversely proportional to the spatial-domain OCT A-line signal-to-noise ratio $SNR_z$.

Even for a structure with uniform macroscopic reflectivity, the A-line magnitude $|h(z, t)|$ will exhibit spatial variation called speckle. Speckle is due to constructive and destructive interference from many scatterers within the coherence length and spot size of the OCT beam. Speckle manifests as granular patterns B-mode images formed from OCT A-line data. Because of speckle, a macroscopically uniformly reflecting structure will give rise to a spatially noisy A-line and there will be some regions that have very low measured intensity due to destructive interference giving rise to a low $SNR_z$ and therefore a high phase noise variance $\sigma_t^2$. If an estimate is made of the motion of a rigid structure spanning multiple regions, the inclusion of the contribution from low SNR regions may reduce the accuracy of the estimate since they contribute significant noise to the estimate without contributing significant signal.

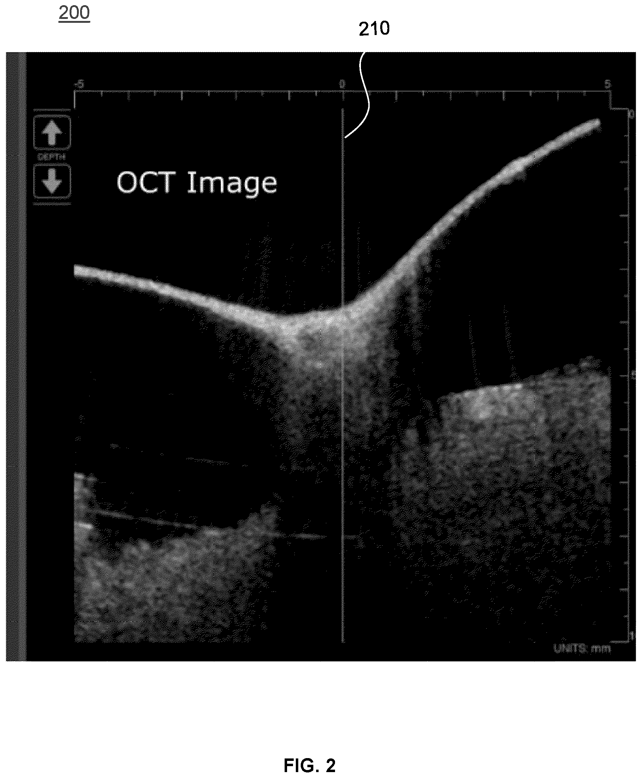

FIG. 2 illustrates an optical coherence tomography image 200 with associated speckle noise, according to some embodiments. The image 200 depicts a cross-sectional B-mode image of the tympanic membrane and the umbo of the malleus.

The malleus is an example of a rigid structure for which a composite vibrometric measure can be obtained. As can be seen in FIG. 2, the OCT structural data (shown here as the intensity of the A-line voxels) includes a mottled structure that results from speckle that spatially varies throughout the image. Accordingly, when a set of interferograms are acquired along a selected depth axis 210 that corresponds to a selected lateral scanning position of the OCT beam, the transformed A-line intensity values, at different voxel locations in the depth domain, will have intensity levels that vary, in part, according to the spatial speckle pattern along the depth axis. Voxels residing within a region of destructive interference will have low intensities, leading to a high phase noise variance $\sigma_t$ for the voxel, leading to noise in the calculation of a vibrometric measure that includes the noisy phase signal from the voxel.

While FIG. 2 shows the spatial impact of speckle at a moment in time, when imaging in live patients, the speckle pattern will change with time due to relative motion between the optical coherence tomography device and the rigid structure being interrogated. This is especially true in applications in which the optical coherence tomography device is a handheld probe that is not precisely positioned in space and can move due to hand motion of the user. Relative motion between the optical coherence tomography device and the rigid structure being measured also occurs due to patient motion including involuntary motion associated with breathing and heartbeat. Accordingly, the speckle pattern changes and moves, relative to the rigid structure, as a function of time.

While various signal processing strategies are known for the mitigation of speckle in OCT images, the present inventors sought a method that could achieve significantly improved SNR in the estimate of a time-dependent optical phase signal, extracted from a set of A-lines, from Doppler OCT vibrometry, that is encoded with the vibrometric response. In cases in which the structure that is optically and acoustically interrogated via Doppler OCT vibrometry is a rigid structure, then motion of the rigid structure, responsive to the acoustic stimulus, could be treated as motion of a single object having a single bulk complex dynamic displacement, and characterized by a single time-dependent phase profile. In such a case, a vibrometric measure associated with the rigid structure could be obtained by combining (e.g. by averaging) contributions from voxels within the rigid structure, leading to an overall improvement in the SNR of the vibrometric measure, when compared to vibrometric measures computed on a per-voxel basis or to vibrometric measures that apply an equal-weighted average across voxels. Such an approach would involve identification (e.g. via segmentation) of the voxels corresponding to the rigid structure, e.g. via the processing of structural OCT data (e.g. the amplitude of the A-line data), and the averaging of vibrometric data from these voxels.

Although such an approach may potentially provide a benefit in terms of reduced noise and increased accuracy, the present inventors realized that this benefit might be nullified or reversed if the contributions of vibrometric data from voxels residing in region of low intensity due to speckle contaminated the estimate, owing to their high phase variance. The present inventors realized that it would be possible to address this problem by generating the vibrometric measure for the rigid structure in such a way that contributions to the vibrometric measure from regions experiencing destructive interference and/or low A-line intensity (i.e. low $|h(z, t)|^2$) are non-uniformly weighted - that is to say - not merely averaged with other regions - but given less weight than contributions from other regions within the rigid structure experiencing constructive interference and high A-line intensity.

Such a non-uniformly weighted approach may result in an improved SNR for the vibrometric measure because it would minimize the contribution of those regions that contribute most to the noise while maximizing the contribution of those regions that contribute most to the signal. Indeed, it was hypothesized that if the contributions to the vibrometric measure from different subregions (e.g. voxels or groups of voxels) of the rigid structure were non-uniformly weighted or incorporated according to their local depth-domain intensity (i.e. to the local depth-domain estimate of $|h(z, t)|^2$), then the contribution to the vibrometric measure from depth subregions having a locally poor SNR due to destructive interference could be suppressed, at least in part, leading to an overall improvement of the SNR of the vibrometric measure characterizing the vibrometric response of the rigid structure.

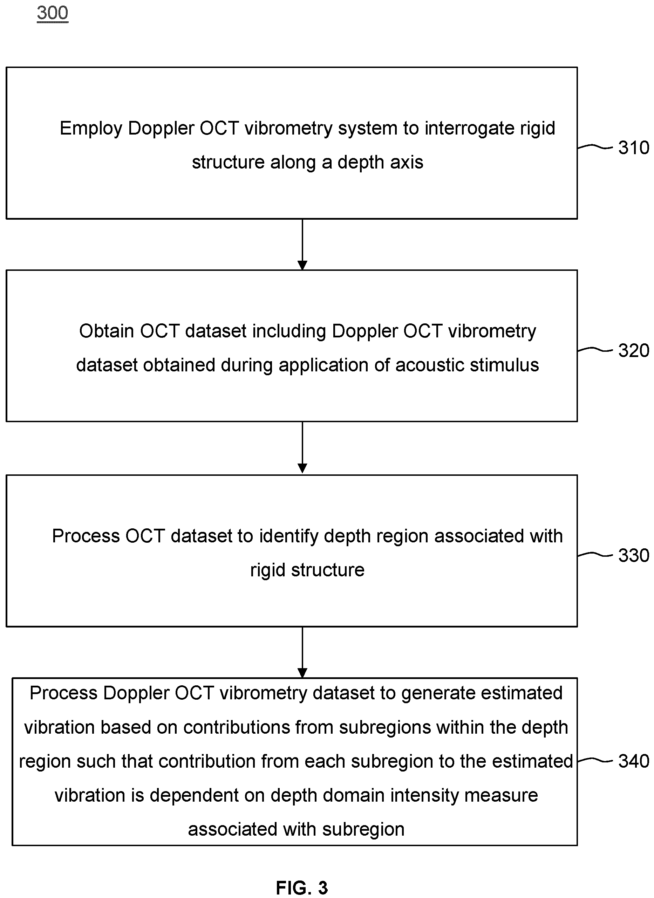

FIG. 3 illustrates a method 300 for performing Doppler OCT vibrometric measurements of acoustic vibrations with reduced susceptibility to speckle noise, according to some embodiments. The method 300 compensates for destructive speckle when generating a composite vibrometric measure associated with a rigid structure.

In 310, a Doppler optical coherence tomography vibrometry system, such as, but not limited to, the system 100 illustrated in FIG. 1, may be employed to interrogate a rigid structure, along a depth axis (e.g. an image line).

In 320, an optical coherence tomography dataset may be obtained that includes at least an optical coherence tomography Doppler vibrometry dataset, where the Doppler optical coherence tomography vibrometry dataset is obtained while applying an acoustic stimulus and includes a plurality of interferograms obtained at different values of the phase of the acoustic stimulus, as described herein. Each interferogram is transformed, for example via an inverse discrete Fourier transform (DFT), to obtain a corresponding A-line that resides in the depth domain (i.e. has values corresponding to depth voxels) and represents the depth dependence of the complex reflectivity profile of the rigid structure, with the vibrometric response of the rigid structure encoded onto the phase data. As described herein, each A-line corresponds to a separate time point, enabling the construction of a dataset, in the time-depth domain, that characterizes the optical phase, both in depth (via the DFT) and in time (via the multiple interferograms) while the acoustic stimulus is applied. The optical coherence dataset may also optionally include a structural optical coherence dataset obtained in the absence of application of the acoustic stimulus. Such a dataset does not have a time dependence and characterizes the static structural response of the rigid structure.

In step 330, the optical coherence tomography dataset may be processed to identify a depth region, residing along the depth axis, that is associated with the rigid structure. In cases in which the optical coherence tomography dataset is only made up of the Doppler optical coherence tomography vibrometry dataset, the Doppler optical coherence tomography vibrometry dataset may be processed to determine the depth region that corresponds to the rigid structure, based on the amplitude of the A-line data, upon which the structural properties of the rigid structure is encoded. Alternatively, if the optical coherence tomography dataset also includes a structural optical coherence tomography dataset such as a B-mode OCT image, the latter can be processed to determine the depth region corresponding to the rigid structure.

It will be understood that there are many methods available to process optical coherence tomography data to identify the depth region (e.g. the set of voxels) that correspond to the rigid structure. In some embodiments, the rigid structure is identified (and optionally segmented) based on its A-line intensity $|h(z)|^2$. In some embodiments, the rigid structure can be identified, at least in part, based on a known shape. In some embodiments, the optical phase, as determined from the Doppler optical coherence tomography vibrometry dataset, may be employed to identify the rigid structure, since all voxels of the rigid structure (such as an ossicle) will have a coherent vibration phase, and thus enabling the optical phase to be used to segment out the rigid structure. In some embodiments, segmentation of the rigid structure may include the use of a smoothing filter and/or the use of a peak finding function to identify proximal and distal bounds of the rigid structure.

Identification and segmentation may be implemented, for example, using one of more segmentation algorithms that are well-known. Such algorithms may involve one or more of thresholding based on one or more of the properties described above, clustering, compression, histogramming, edge detection, region growing (e.g. through erosion and dilation methods), methods based on minimization of a cost function such as parametric methods, level set methods and marching methods, variational methods, graph partitioning methods, watershed methods, model-based methods such as those that rely on distortion of a template.

In some embodiments, by involving the segmentation of bony structures, the segmentation algorithm may be configured to search for the most distal structure visible along a given image line, since a bony structure, if thicker than approximately 1mm will shadow more distal objects such that no reflected light is received from them. In other words, since it is not possible to obtain OCT light from depths beyond a bony structure, identification of a bony structure along a path containing multiple structures can be performed by identifying the distalmost structure within the structural OCT data.

Moreover, prior to various processing steps disclosed below, one or more additional preprocessing steps may be performed, such as, but not limited, to, depth truncation of the dataset, removal of background fixed pattern artefacts or deconvolution of the axial point spread function.

In step 340, the optical coherence tomography Doppler vibrometry dataset is processed to generate one or more vibrometric measures characterizing a vibrometric response of the rigid structure to the acoustic stimulus. As explained above, the optical phase $\phi(z, t)$ of the A-line (the transformed interferogram) is encoded with the vibrometric response $\phi_{vib}$

($z, t$) = $2k_0\delta z(t)$ of the rigid structure. Time-dependent optical phase data is therefore obtained from the plurality of A-lines, and this optical phase evolves with the frequency of the applied stimulus. To obtain a continuous optical phase free from discontinuities of whole integer multiples of $2\pi$ radians, phase unwrapping may be performed as well.

The vibrometric measure is obtained by processing the portion of the optical coherence tomography Doppler vibrometry dataset that is associated with the rigid structure, as determined by step 320 of the method 300 shown in FIG. 3. In other words, the optical coherence tomography Doppler vibrometry data within voxels associated with the rigid structure is processed to determine the vibrometric measure.

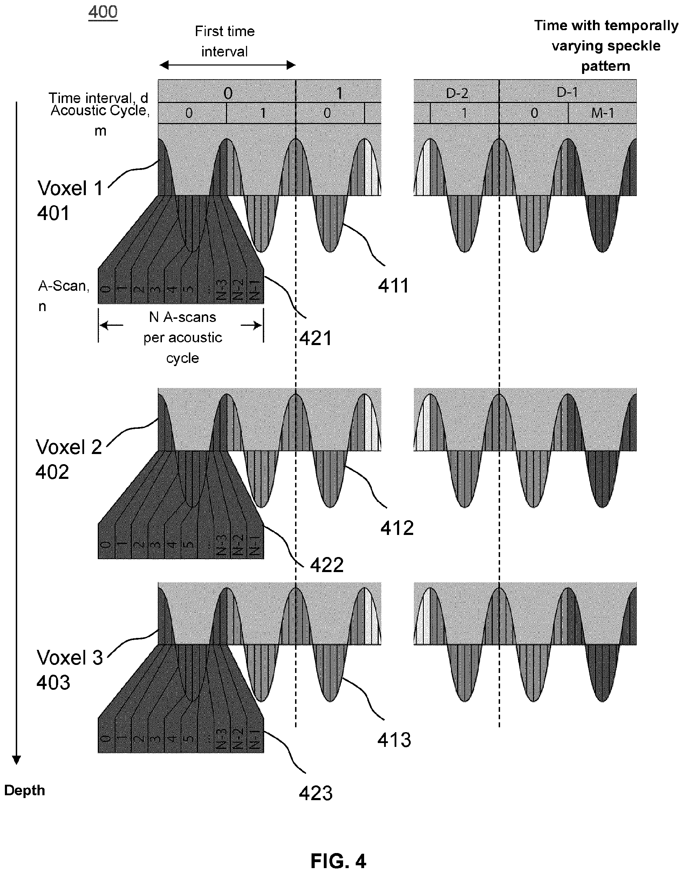

FIG. 4 illustrates a system 400 of the time dependence of the optical phase extracted from the A-lines, according to some embodiments. The set of acquired interferograms are transformed into a corresponding set of A-lines, each A-line having a depth dimension, and an unwrapped optical phase value is obtained for each A-line. FIG. 4 illustrates a temporal phase evolution (411, 412, 413) of three example voxels (401, 402, 403) along the depth direction (e.g. the first three voxels among a set of voxels determined to correspond to the rigid structure in step 330 of FIG. 3) relative to the timing of the acoustic cycles.

As shown in FIG. 4, each voxel has an associated optical phase time series (with one point per A-line) that is extracted from the set of A-lines (421, 422, 423), with the optical phase (411, 412, 413) evolving at the frequency of the acoustic stimulus, encoded with the vibrometric response of the rigid body. Although the optical phases of the three pixels are shown as being aligned (in phase), it will be understood these phases will generally be offset from one another due to the different depth-dependent static phase offsets associated with the rigid structure. The static optical phase offset may be subtracted from the measured optical phase by subtracting the optical phase measured at the same time point at each depth from the optical phase measured at all time points at the corresponding depths, or by calculating the average phase over a period of time and subtracting that from all phases.

Since the motion of the rigid structure, responsive to the acoustic stimulus, can be treated as motion of a single object having a bulk motion, characterized by a single time-dependent phase profile, the set of optical phase time series (411, 412, 413) from the voxels associated with the rigid structure can be employed to generate a composite estimate of the vibrometric measure.

As some of the voxels will have a low A-line intensity $|h(z, t)|^2$ and therefore high phase noise variance $\sigma_t^2$ due random destructive interference associated with speckle, the contribution of such voxels to the calculation of the vibrometric measure can be reduced relative to the contribution from voxels with higher intensity and lower phase noise variance, thereby leading to a vibrometric measure that characterizes the vibrometric response of the rigid structure with less noise than that which would be achieved with equal-weighted averaging of the measured optical phase from all pixels.

While some embodiments may involve incorporating non-uniformly weighted contributions on a per voxel basis, it will be understood that contributions can, more generally, be incorporated on a per-subregion basis, where each subregion includes at least one voxel, and where a given subregion may include two or more voxels.

Accordingly, as illustrated in step 340 of FIG. 3, a vibrometric measure may be generated based on contributions from at least one subregion within the depth region associated with the rigid structure, such that the contribution made to the vibrometric measure, from each subregion, is dependent on a depth-domain intensity measure associated with the subregion. Such an approach facilitates the calculation of the vibrometric measure with improved accuracy and less susceptibility to uncertainty from speckle noise.

In some embodiments, the incorporation of non-uniformly weighted contributions is included, within the spatial domain, of one or more subregions associated with the rigid structure, according to the local depth-domain A-line intensity associated with each subregion. Such an approach can be beneficial in reducing contributions to the vibrometric measure, from subregions that are associated with reduced A-line intensity due to destructive speckle, resulting in an improved estimate of the vibrometric measure.

Accordingly, in some embodiments, each subregion is chosen to be sufficiently small to encompass speckle-induced spatially-dependent changes in depth-domain intensity within the depth region associated with the rigid structure. For example, each subregion may be the size of an individual A-line voxel, or, for example, a subregion may be made up of a plurality of voxels, provided that the subregion is smaller than a typical speckle grain size, such as, for example, having an extent, in the depth direction, of less than 50 microns.

The method 300 may also be adapted to reduce the impact of temporal variations of the speckle pattern on the estimated vibrometric measure. Indeed, as described herein, the speckle pattern moves and changes temporally due to changes in the relative positioning of the Doppler OCT vibrometric device and the rigid structure. If the extracted optical phase time series are segmented into a series of time intervals, then an A-line intensity measure associated with each time interval can be employed such that the contribution to the vibrometric measure, from each subregion (depth domain) and each time interval (time domain) is dependent on a respective intensity measure associated with each subregion and time interval.

Each time interval may be selected to be sufficiently short to encompass speckle-induced time-dependent changes in depth-domain intensity within the one or more subregions due to relative motion between the optical coherence tomography Doppler vibrometry system and the rigid structure. For example, the geometric relationship between the device and the rigid structure is typically changing with time and it may only take 50 microns of motion to completely decorrelate the speckle pattern. Accordingly, drift of the hand during a vibrometric measurement (e.g. when performing step 310 of FIG. 3) may completely decorrelate the speckle pattern so that a pixel that previously had a low intensity might now have a high intensity and vice versa. The timescale over which this decorrelation of the speckle-induced pixel intensity occurs is the timescale over which the image moves by either a coherence length axially or a coherence radius laterally or in which the angle of the beam changes by the wavelength divided by the coherence radius.

For example, if the user moves their hand at $v = 1cm/s$ (a typical speed for wrist motion) and the beamwidth is w = $50\mu m$, the speckle pattern will rearrange itself over a timescale $t_{coh} = \frac{w}{v}$ of approximately 5ms.

Referring again to FIG. 4, it can be seen that each optical phase time series is broken up into a series of time intervals. An A-line intensity measure can be calculated for each time interval of each voxel (e.g. an average of the A-line intensity $|h(z, t)|^2$ over the time interval), such that each time interval of each voxel has an associated A-line intensity measure. The vibrometric measure may then be computed such that the contributions from each pixel-(time interval) combination are dependent on the respective intensity measures associated with each pixel-(time interval) combination. While FIG. 4 illustrates each time interval as corresponding to two complete cycles of the acoustic phase, it will be understood that this temporal segmentation is intended merely as a non-limiting example and that in other embodiments, each time interval may include a different number of acoustic cycles (whole or fractional).

Accordingly, various methods of the present disclosure employ the non-uniform weighting of contributions to the vibrometric measure based on the intensity of the A-line across depth, and the dynamic temporal updating of the weighting across time to compensate for the impact of motion on the speckle pattern. In some embodiments, the temporal weighting is updated faster than the timescale over which the speckle changes.

In some embodiments, the present methods may be adapted to include one or more processing steps that remove or reduce motion noise. For example, methods disclosed in International Patent Application No. PCT/CA2018/050200 may be employed to reduce a contribution of motion noise, prior to performing the calculation of the vibrometric measure associated with the rigid structure. In some cases, the vibrometric measure and the phase noise can be modeled with a parametric model that incorporates parameters describing the vibrometric measure and parameters describing the noise. For example, a parametric model in which phase noise is modeled as being linear on the time-scale of a single acoustic cycle and in which the acoustic stimulus is sinusoidal, the vibrometric dynamic displacement can be modeled as:

$$\hat{\phi}\left(t = \frac{n}{f_s}\right) = \frac{4\pi A_z}{\lambda_0} \sin\left(\frac{2\pi n}{N} + \phi_0\right) + cn + d \quad (5)$$

where $n$ indexes the laser sweep number, $A_z$ is the vibration amplitude, $\phi_0$ is a constant phase offset, N is the number of laser sweeps per acoustic cycle, and $f_s$ is the sweep rate and c and d describe the slope and offset of the noise when modeled as an additive, linear contribution to the measured displacement.

To apply the linear least squares method to the determination of the parameters in the above equation, the sinusoidal term can be broken into two orthogonal components rather than a sine function with a phase offset, resulting in a function of sines and cosines in the following form:

$$\hat{\phi}\left(t = \frac{n}{f_s}\right) = \frac{4\pi \widetilde{A_z}}{\lambda_0} \cos\left(\frac{2\pi n}{N}\right) + \frac{4\pi \widetilde{B_z}}{\lambda_0} \sin\left(\frac{2\pi n}{N}\right) + cn + d \quad (6)$$

where $\widetilde{A_z}$ and $\widetilde{B_z}$ are parameters describing the in-phase and quadrature components of the displacement. This method can advantageously be performed on a per-time-interval basis to remove phase errors due to motion artifacts. Indeed, such an approach breaks the drift that will occur over the measurement into linear slices, enabling the removal of the linear and the offset terms, leaving just sinusoidal terms that are encoded with the vibrometric response.

The method 300 may be implemented and/or adapted according to many different embodiments, and various non-limiting examples of such embodiments are described herein.

It will be understood that the contribution associated with a given subregion (e.g. voxel or group of voxels) may be determined according to a wide range of embodiments. For example, the contribution may be generated according to a weight that is applied, on a per-subregion basis, where the weight is calculated based on the intensity of the A-line $|h(z, t)|^2$ associated with the subregion. The intensity may be a measure of central tendency of the intensity over a given time duration, such as a total time duration over which the interferograms are acquired for generating the vibrometric measure, or, for example, a subset of this time, as illustrated in FIG. 4 and described in further detail herein.

Weighted averaging is one such method of estimating the expectation value of a signal (i.e. a measure of central tendency) from a set of samples where the noise variance for each sample is different. In such situations, an inverse variance weighted average provides an optimal estimate of the expectation value. As previously discussed, the vibrometric phase signal $\phi(t, z)$ is contaminated by additive noise of variance $\sigma_t^2 = \frac{1}{2SNR_z}$ and so an inverse variance weighting for the phase will include weighting by $\frac{1}{\sigma_t^2} = 2SNR_z$.

If the noise $n(k, t)$ on the interferogram is Gaussian and is independent and identically distributed with respect to the wavenumber $k$, then, following Fourier transformation, it will give rise to noise $n(z, t)$ in the spatial domain that is independent and identically distributed with respect to $z$. If, additionally, the noise is stationary, then its statistics will also be independent of time. Thus, for any $t$ and depth $z$, the variance $\sigma_n^2$ of the additive noise $n(z, t)$ contaminating the complex OCT A-line signal $h(z, t)$ is a constant independent of $t$ and $z$. According to the previous discussion, the variance of the noise on the acoustic phase signal at a depth z and time t is given by:

$$\sigma_t^2[z, t] = \frac{1}{2SNR_z} = \frac{\sigma_n^2}{2|h(z,t)|^2}. \qquad (7)$$

Because $h(z, t)$ will vary with time $t$ and depth $z$, $\sigma_t^2$ also varies with time and depth even though $\sigma_n^2$ does not. If, for each depth z, within a rigid structure, the signal is divided into a set of M time intervals $mT$ where $T \ll t_{coh}$, a set of estimates $x_{m,z}$ are made of a vibrometric measure X for each time interval $mT$ and depth z. An overall estimate of the vibrometric measure $\hat{x}$ for a rigid structure can be obtained as a weighted average of estimates for each time interval and depth such that $\hat{x} = \Sigma_z\Sigma_m w_{m,z}x_{m,z}$ where $w_{m,z}$ are weights at each time interval and depth. The weights are constrained to be normalized such that $\Sigma_z\Sigma_m w_{m,z} = 1$.

The inverse variance weighted average is obtained when the weights are made proportional to the inverse of the variance of the phase noise, i.e. when $w_{m,z} = \frac{1}{W}\frac{1}{\sigma_t^2[z,m]}$ where $\sigma_t^2[z, m]$ is the phase variance for time interval $mT$ and depth z and where $W = \Sigma_z \Sigma_m \frac{1}{\sigma_t^2[z,m]}$ is a normalization factor that ensures $\Sigma_z\Sigma_m w_{m,z} = 1$. Since $T \ll t_{coh}$, $h(z, t)$ does not change significantly during one period $T$ and so $|h(z, m)|^2$ can be estimated from a measure of central tendency of $|h(z, t)|^2$ over the period, e.g. from the average of $|h(z, t)|^2$ over the period $|h(z, m)|^2 = \int_{mT}^{(m+1)T} |h(z, t)^2| \, dt$. Thus the weights are:

$$w_{m,z} = \frac{1}{W}\frac{1}{\sigma_t^2[z,m]} = \frac{1}{W}\frac{2|h(z,m)|^2}{\sigma_n^2} = \frac{|h(z,m)|^2}{\Sigma_z\Sigma_m|h(z,m)|^2}. \qquad (8)$$

With these weights, the estimate $\hat{x}$ of the vibrometric measure X for the rigid structure as a whole across depth and time is:

$$\hat{x} = \Sigma_{m,z} w_{m,z}x_{m,z} = \Sigma_{m,z} \frac{|h(z,m)|^2}{\Sigma_z\Sigma_m|h(z,m)|^2} x_{m,z}. \qquad (9)$$

It will be understood that the example above provides one non-limiting example of the calculation of a weighting, and that other weightings that preferentially weight high A-line intensity depth regions and/or time intervals may be calculated in the alternative. In cases in which weights are recomputed during a measurement interval, it is beneficial to update the weights at a rate that is fast enough to compensate for speckle variations caused by hand motion, i.e. in a time $T \ll t_{coh}$.

While some embodiments involved the calculation of per-subregion (e.g. per-voxel) weights for non-uniformly weighing the contributions made to the calculation of the vibrometric measure, it will be understood that there are many alternative ways in which the contribution from each subregion can be dependent on a depth-domain intensity measure associated with the subregion.

In some embodiments, instead of weighing the contributions from each subregion (e.g. voxel) based on intensity (energy - e.g. the square of the A-line amplitude at a given depth and time), a subset of subregions (for example, a single voxel) that satisfy pre-established criteria may be selected for inclusion in generating the vibrometric measure, or a subset of subregions (for example, a single voxel) that satisfy a statistical criteria (e.g. representing or approximating a mean or median intensity) may be selected for inclusion in generating the vibrographic measure. Examples of such pre-established criteria include voxels whose $SNR_z$ exceeds a defined threshold, voxels whose A-line intensity exceeds a threshold, the set of voxels whose A-line intensity lies within a percentile, a fixed number of voxels with the highest A-line intensity from among all voxels, a set of voxels with the lowest phase noise over a defined time period, and the voxel whose phase is a defined percentile (e.g. the median) of the phase of all voxels.

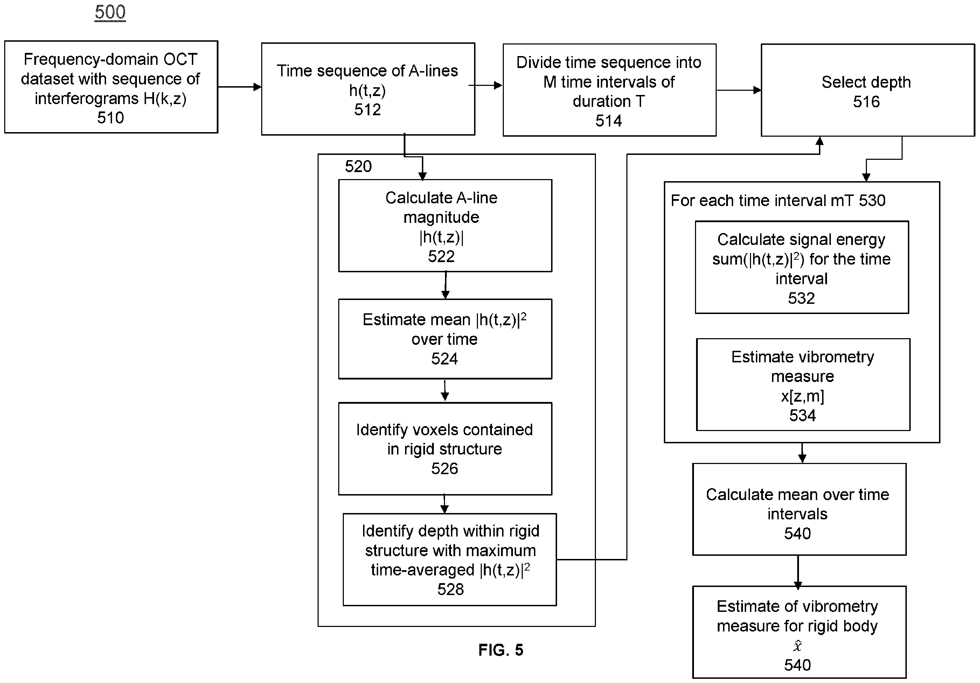

FIG. 5 illustrates an algorithm flowchart 500 for calculating a vibrometric measure from a Doppler OCT vibrometry dataset using a single pixel fit algorithm, according to some embodiments. The algorithm flowchart 500 may include where the vibrometric measure associated with a rigid structure may be computed based on a depth-domain intensity measure associated with a specific subregion.

Steps 510-516 may be performed sequentially. In 510, the frequency domain OCT dataset may be provided. The OCT dataset may include a sequence of interferograms H(k,z). In 512, a time sequence of A-lines h(t,z) may be accepted, which are obtained from the frequency domain OCT dataset in 510. In 514, the time sequences from 512 may be divided into M time intervals, each of duration T. That is, the measured A-lines may be divided temporally into M time intervals, where in one non-limiting example, each time interval is one acoustic period long. In 516, a depth may be selected. The depth may correspond to the depth region of a corresponding rigid structure and may be the voxel with the brightest average A-line intensity over time. The depth may be determined based on 520. The steps in 520 may be performed sequentially.

In 520, the depth may be determined based on identified voxels in the rigid structure. In 522, the A-line magnitude of a voxel may be calculated. In 524, the estimation of the vibrometric measure on the one voxel may be identified as the vibrometry estimate mean for the entire rigid structure. In 526, based on the estimated mean calculated in 524, voxels contained in the rigid structure may be identified. In 528, the depth within the rigid structure may be identified. The depth may include the depth with the highest time-averaged A-line magnitude. This depth from 528 is then added to the selected depth of 516.

In 530, for each time interval mT, the complex vibration amplitude may be estimated. In cases where the vibration is excited by a sinusoidal stimulus, the estimation may be obtained by applying a DFT and selecting the complex component of the DFT at the stimulus frequency. Alternatively, if a parametric model is available to describe the expected vibrometric response of the system to a stimulus, a parametric fit may be performed to the model to estimate the parameters which may include parameters describing the complex vibration amplitude and phase.

Such parametric models may include terms that estimate the contribution of noise sources to the response like the models described by equations (5) and (6). In 532, the signal energy sum for the time interval may be calculated. In 534, based on the signal energy sum calculated in 532, the vibrometry measure may be estimated. In 540, the mean over time intervals may be calculated. Steps 532 and 534 may be performed in parallel.

In 550, the vibrometric measure for the rigid structure may be estimated. The selection of the voxel with the brightest average intensity over time suppresses contributions from voxels that, on average, suffer most heavily from destructive interference due to speckle and so improves SNR as compared to estimates that include these voxels. In effect, the algorithm flowchart 500 performs a very discrete form of depth weighting, in which the pixel with the average brightness is weighted at unity, and all other pixels at zero. The algorithm flowchart 500 applies uniform weighting in the temporal dimension.

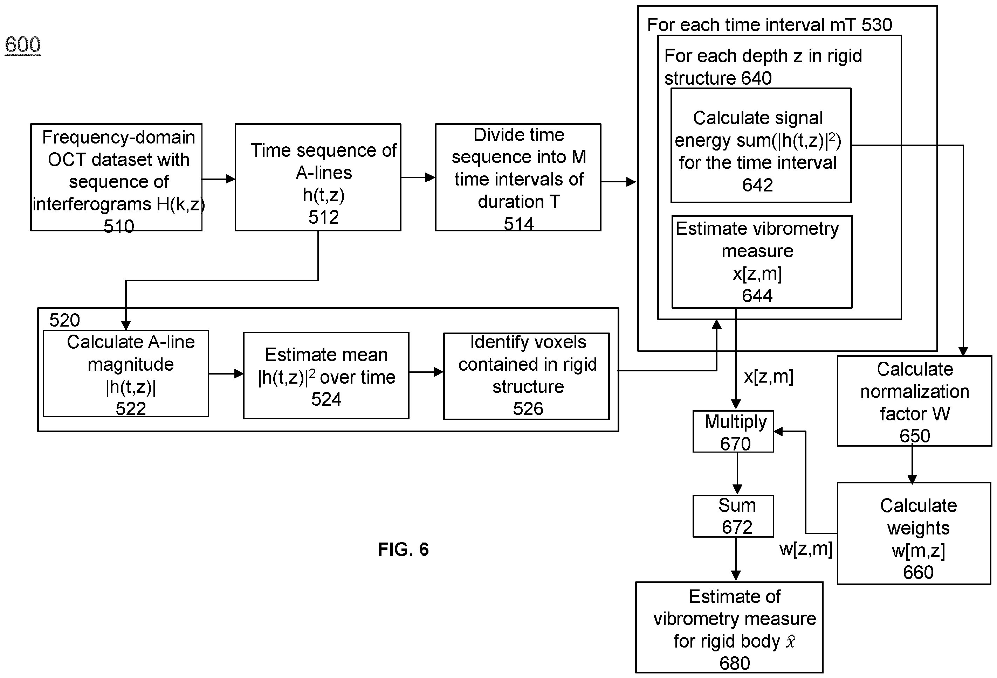

FIG. 6 illustrate an algorithm flowchart 600 for calculating a vibrometric measure from a Doppler OCT vibrometry dataset using a fitted brightness weighted (inverse variance weighted) algorithm, according to some embodiments. The algorithm flowchart 600 incorporates both per-voxel and per-time-interval weights when computing a vibrometric measure associated with a rigid structure.

As in FIG. 5, steps 510-514, which may be performed sequentially, are also in algorithm flowchart 600, with the A-lines calculated at all time and depth values, as shown at steps 510-514. In 510-514, a frequency domain OCT dataset is provided including a sequence of interferograms H(k,z), which are converted to a time sequence of A-lines h(t,z). The sequence of A-lines 512 may be divided into M time intervals, each of duration T.

Additionally, as in FIG. 5, in 520, the depth may be determined based on identified voxels in the rigid structure and used in 630. In 522-526, which may be performed sequentially, the A-line magnitude of a voxel may be calculated, the estimation of the vibrometric measure on the one voxel may be identified as the vibrometry estimate mean for the entire rigid structure, and based on the estimated mean calculated in 524, voxels contained in the rigid structure may be identified. The A-lines may then be divided into time intervals for all voxel depths (in the present non-limiting example, each containing one acoustic period).

In 630, for each time interval mT, and in 640, for each depth z in the rigid structure, depths corresponding to the rigid structure are employed and the signal energy sum for the time interval may be calculated in 642. In 644, based on the signal energy sum calculated in 642, the vibrometry measure may be estimated. The resulting vibrometric measures in 642, such as the complex displacement (or other example vibrometric measures such as those described below) are multiplied by the A-line intensity $|h(z, m)|^2$ calculated on the A-lines corresponding to the time intervals indexed by $m$. The vibrometric measures estimated on individual depth regions and time intervals are weighted by the normalized A-line intensity $\frac{|h(z,m)|^2}{\sum_z \sum_m |h(z,m)|^2}$ and added together, resulting in an improved estimate of the vibrometric measure. Steps 642 and 644 may be performed in parallel for each time interval and each depth in the rigid structure.

An estimate of the complex vibration amplitude is made for each time interval using either a non-parametric (e.g. DFT-based) method or a parametric method (e.g. model fitting as described above). The estimate of the vibrometric measure is more accurate than the estimate that includes a single pixel both because spatially uncorrelated noise averages out in the depth direction and because at each time interval, contributions from voxels with low phase noise are weighted more heavily than those with high phase noise due to the intensity-based weighting.

In 650, a normalization factor W may be calculated based on the signal energy sum from 642. In 660, weights may be calculated based on the normalization factor in 650. The per-voxel and per-time-interval weights may be calculated and used to weigh the contribution of estimates of vibration measure in each voxel-(time interval) when calculating the composite vibrometric measure for the rigid structure. The weights may be used in 670.

In 670, the estimated vibrometry measure calculated in 644 may multiplied by the weights calculated in 660. In 672, the weighted sum may then be calculated. In 680, the vibrometry measure for a rigid body may be estimated based off the weighted sum in 672.

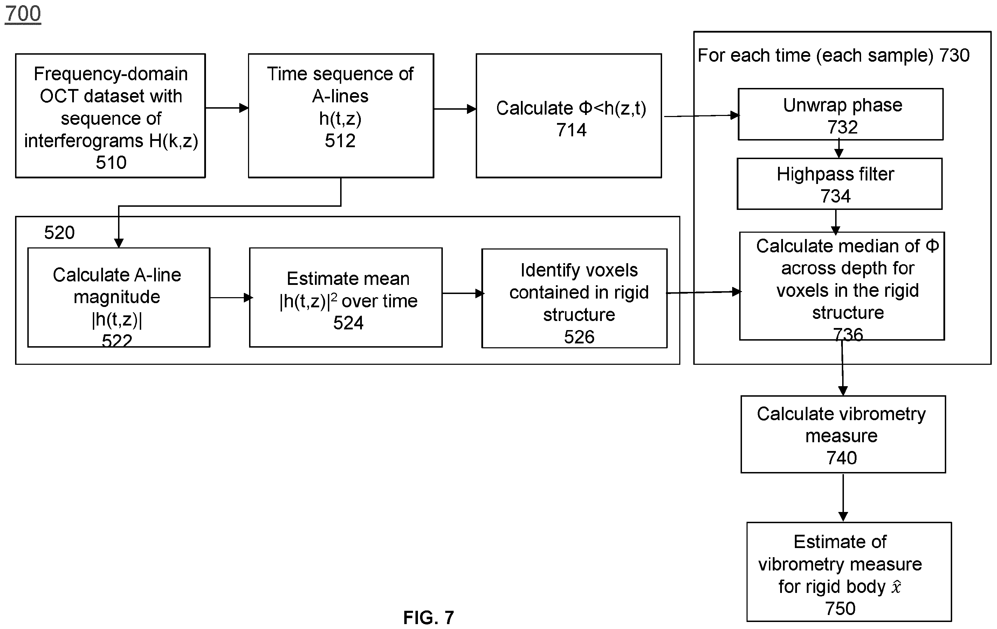

FIG. 7 illustrates an algorithm flowchart 700 for calculating a vibrometric measure from a Doppler OCT vibrometry dataset using a phase median algorithm, according to some embodiments. While intensity-weighted averaging is an effective method for ensuring that voxels with low phase noise contribute most strongly to the estimate of overall motion of a rigid structure, other, less explicit forms of SNR-based weighting can also improve the accuracy of vibration measure estimation such as the algorithm flowchart 700.

In algorithm flowchart 700, the phase may be calculated for all times at all depths associated with the rigid structure and the phase is unwrapped along the time dimension. As in FIGS. 5-6, steps 510-512 are also in algorithm flowchart 600 and 700, with the optical phase of the A-lines is extracted for all time and depth values. In 510-512, which may be performed sequentially, a frequency domain OCT dataset is provided including a sequence of interferograms H(k,z) used to calculate a time sequence of A-lines h(t,z), which may be obtained from the frequency domain OCT dataset. In 714, the A-line phase may be calculated.

Additionally, as in FIGS. 5-6, in 520, the depth may be determined based on identified voxels in the rigid structure and used in 630. In 522-526, which may be performed sequentially, the A-line magnitude of a voxel may be calculated, the estimation of the vibrometric measure on the one voxel may be identified as the vibrometry estimate mean for the entire rigid structure, and based on the estimated mean calculated in 524, voxels contained in the rigid structure may be identified.

In 730, for each time and each sample, the phase calculated in 714 may be unwrapped in 732 and the resulting time-dependent phase traces filtered in time 730 to eliminate or suppress low frequency bulk motion. Based on the filtered, unwrapped phase in 734, in 736 the median of the phase may be calculated across the depth of each voxel in the rigid structure. In 736, the median phase over depth is calculated at each time point (i.e. at each laser sweep). Because achieving a high degree of destructive interference requires alignment of a large number of random phases, voxels with very low $SNR_z$ are comparatively rare and form a minority of voxels within a rigid structure. This minority of voxels will exhibit high phase noise and so will typically deviate significantly from the majority of voxels that have low phase noise. Thus, the median phase will almost always arise from a high SNR, low noise voxel, and the selection of the median phase at each time point will provide an accurate estimate of the vibrational response of the rigid structure at that time point without the need for explicit weighting by SNR. A similar benefit may be obtained by selecting a percentile other than the median (e.g. the first quartile) and by calculating the median over a time interval longer than a single laser sweep.

In 740, the vibrometry measure may be calculated based on the median phase over depth calculated in 736. In 750, the vibrometry measure for a rigid body may be estimated. Although the method shown in FIG. 7, and variations thereof, are configured such that the contributions of the subregions are evaluated based on depth distribution of the optical phase, such methods implicitly preferentially apply a higher weight to subregions of higher intensity, since phase outliers that are rejected by the present example methods will be associated with subregions of low intensity. It will be understood that a wide range of vibrometric measures may be computed according to the embodiments disclosed herein. In some non-limiting embodiments, one or more vibrometric measures may include a displacement amplitude, a displacement phase, a complex displacement, velocity amplitude, velocity phase, complex velocity, complex acceleration, acceleration magnitude, acceleration phase and ratios of these measures to a driving pressure or force that produce compliance, stiffness, immittance, admittance, impedance, accelerance, or mobility. Velocity may be calculated, for example, by multiplying the displacement amplitude by 2pf, where f is the frequency of the applied acoustic stimulus.

It will be understood that the vibrometric measures may be generated as vibrometric images, or as non-image data. For example, one or more vibrometric measures may be generated based on the measurement at a single image line, or, for example, at a subset of image lines that correspond to different structures. Furthermore, although sinusoidal excitation provides magnitude and phase at each voxel, these could be used to construct more generic measures that may be of diagnostic importance or utility. For example, comparative measures, such as the ratio of umbo to stapes motion may be a good indicator of ossicular fixation, and this measure can be calculated using vibrational phase data as an input.

The present disclosure provides systems and methods useful for performing Doppler optical coherence tomography vibrometry for assessment of pathologies of the middle ear, the inner ear and the larynx. It may also be useful for performing elastographic measurements in which anatomy that does not vibrate under normal physiological conditions is driven to vibrate and the vibrational response is measured to determine the mechanical compliance of tissue. In some embodiments, the systems and methods may be employed for accurate assessment of mobility associated with one or more rigid structures in the ear, such as, for example, rigid bony structures in the ear such as the ossicles (malleus, incus and stapes), the tympanic membrane, soft tissue or foreign bodies located in the middle ear space coupled to the ossicular chain, the round window, oval window, and membranes of the cochlea. The example vibrometric measurement methods of the present disclosure may be employed, for example, in the diagnosis of pathologies such as, but not limited to, otosclerosis, cholesteatoma, atelectasis, cochlear canal dehiscence, glomus tumor, tympanosclerosis, middle ear trauma, conductive hearing loss, perilymphatic fistula, Meniere's disease, congenital malformation of the middle ear, otitis media, ossicular erosion, paragangliomas, Eustachian tube dysfunction, tympanic membrane perforation and failure of prior middle ear surgery, including failure of middle ear implants.

In some embodiments, a single image line (corresponding to a single lateral scanning location) is selected for collecting the Doppler OCT data to generate the one or more vibrometric measures. For example, the scanning subsystem can be controlled such that the dwell time at the selected image line is sufficiently long to permit the acquisition of a sufficient number of interferograms for generating the vibrometric measures with a suitable signal-to-noise ratio, such as a dwell time on the order of seconds. The system can optionally be employed to rapidly scan among a remainder of the image lines (e.g. non-selected image lines), with a dwell time on the order of milliseconds, in order to collect interferograms suitable for generating B-mode image data.

In another example embodiment, vibrometric data may be acquired, for a selected image line, according to a plurality of partial vibrometric data acquisition steps, with the scanning subsystem interrogating other lateral positions in between each partial vibrometric data acquisition step. The set of partial vibrometric data acquisition steps can be processed together to generate one or more vibrometric measures. For example, the scanning subsystem may be configured to scan the OCT optical beam to collect a plurality of image frames, with each image frame involving the collection of data (e.g. structural image data) along a plurality of image lines, where one partial vibrometric data acquisition step, for a selected image line, is performed for each frame, thereby temporally interleaving the collection of partial vibrometric data with the collection of the image frame data. Such an embodiment is beneficial in reducing the time required for vibrometric measurements via the selection of a subset of image lines for vibrometric analysis, while maintaining a higher frame rate (e.g. sufficiently high for real-time B-mode imaging) by permitting averaging of vibrometric measures among multiple image frames.

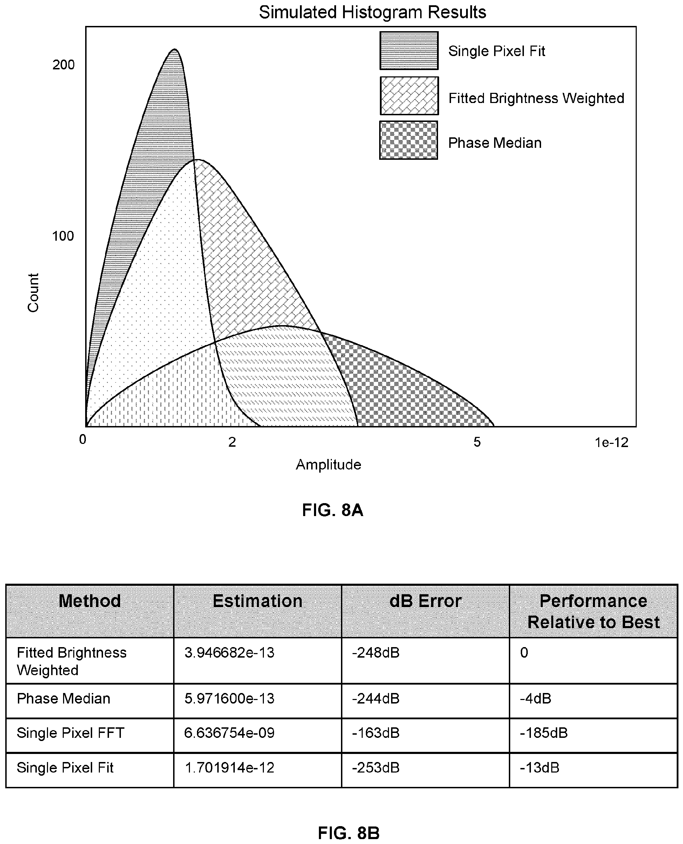

FIGS. 8A and 8B illustrate results of a simulation comparing the estimated vibration amplitude when the true amplitude is zero for the methods illustrated in FIGS. 5-7, according to some embodiments.

The example models shown in FIGS. 5 ("Single Pixel Fit"), FIG. 6 ("Fitted Brightness Weighted"), FIG. 7 ("Phase Median") were simulated to demonstrate that in a signal with known noise statistics the estimate of vibrational amplitude and error are improved in systems employing depth and time weighting (explicitly in FIG. 6 and implicitly in FIG. 7) relative to uniform or trivial weighting (FIG. 5). The simulation results, seen in FIG. 8A, show a histogram of measured vibration amplitudes over 1000 simulated experiments in a situation where the true vibration amplitude is zero, i.e. one in which all apparent vibration is due to noise. The simulation includes effects from time-dependent speckle such that the depth regions exhibiting destructive interference change over the course of each experimental run.

Since the true vibration level is zero, methods that produce estimated vibration clustered close to zero are more accurate than ones that exhibit a wider range of estimated vibration or a mean vibration estimate that deviates from zero.

FIG. 8A shows that methods incorporating intensity-based depth and time weighting, either explicitly (e.g. fitted brightness weighting) or implicitly (e.g. phase median) exhibit better performance than those employing trivial or uniform weighting (e.g. single pixel fit). This result is expected as the trivial weighting in depth of the single pixel methods fails to take full advantage of information in pixels that are not the average brightest pixel and the uniform weighting in time includes times when the average brightest pixel has a low intensity due to variation in the speckle pattern caused by relative motion between the anatomy being interrogated and the handpiece. Additionally, one can observe that the fitted brightness weighted method outperforms the phase median method because it combines estimates at different depths and time intervals in a more optimal manner.

Additionally, FIG. 8A illustrates that the averaging methods may achieve better performance than the single pixel method. This may be expected as the average brightness pixel is not guaranteed to be the brightest pixel for all points of time in the presence of drift due to speckle. Additionally, the optimality of the brightness weighted average against the nearly optimal median method may be observed. FIG. 8B describes the numerical results of the methods of FIG. 8A. For example, the brightness weighted, and median methods may be superior with the difference between those two methods of 4dB.

The specific embodiments described above have been shown by way of example, and it should be understood that these embodiments may be susceptible to various modifications and alternative forms. It should be further understood that the claims are not intended to be limited to the particular forms disclosed, but rather to cover all modifications, equivalents, and alternatives falling within the spirit and scope of this disclosure.

FIG. 9 illustrates a control and processing circuitry/hardware 900 for controlling the Doppler OCT vibrometry system for measurement of one or more vibrometric measures associated with a rigid structure, according to some embodiments. The example control and processing hardware 900 may include a processor 910, a memory 915, a system bus 905, one or more input/output devices 920, and a plurality of optional additional devices such as communications interface 925, external storage 930, and a data acquisition interface 935. In some embodiments, a display (not shown) may be employed to provide a user interface for facilitating input to control the operation of the system 900. The display may be directly integrated into a control and processing device (for example, as an embedded display), or may be provided as an external device (for example, an external monitor).

The control and processing system 900 may include or be connectable to a console that provides an interface for facilitating an operator to control the OCT device. The console may include, for example, one or more input devices, such, but not limited to, a keypad, mouse, joystick, touchscreen, and may optionally include a display device.

The methods described herein, such as methods for controlling acquisition of Doppler OCT vibrometric data and the processing of vibrometric measures, and other example methods described herein, may be implemented via processor 910 and/or memory 915, for example, via executable instructions represented as OCT control module 950, vibrometric processing module 960, DFT processing module 970, and rigid structure segmentation module 980, respectively. Such executable instructions may be stored, for example, in the memory 915 and/or other internal storage.

The methods described herein can be partially implemented via hardware logic in processor 910 and partially using the instructions stored in memory 915. Some embodiments may be implemented using processor 910 without additional instructions stored in memory 915. Some embodiments are implemented using the instructions stored in memory 915 for execution by one or more microprocessors. Thus, the disclosure is not limited to a specific configuration of hardware and/or software.

It is to be understood that the example system shown in the figure is not intended to be limited to the components that may be employed in a given embodiment. For example, the system may include one or more additional processors. Furthermore, on or more components of control and processing hardware 600 may be provided as an external component that is interfaced to a processing device. Furthermore, although the bus 905 is depicted as a single connection between all of the components, it will be appreciated that the bus 905 may represent one or more circuits, devices or communication channels which link two or more of the components. For example, the bus 905 may include a motherboard. The control and processing hardware 900 may include many more or less components than those shown.

Some aspects of the present disclosure can be embodied, at least in part, in software, which, when executed on a computing system, transforms an otherwise generic computing system into a specialty-purpose computing system that is capable of performing the methods disclosed herein, or variations thereof. That is, the techniques can be carried out in a computer system or other data processing system in response to its processor, such as a microprocessor, executing sequences of instructions contained in a memory, such as ROM, volatile RAM, non-volatile memory, cache, magnetic and optical disks, or a remote storage device. Further, the instructions can be downloaded into a computing device over a data network in a form of compiled and linked version. Alternatively, the logic to perform the processes as discussed above could be implemented in additional computer and/or machine-readable media, such as discrete hardware components as large-scale integrated circuits (LSI's), application-specific integrated circuits (ASIC's), or firmware such as electrically erasable programmable read-only memory (EEPROM's) and field-programmable gate arrays (FPGAs).

A computer readable storage medium can be used to store software and data which when executed by a data processing system causes the system to perform various methods. The executable software and data may be stored in various places including for example ROM, volatile RAM, nonvolatile memory and/or cache. Portions of this software and/or data may be stored in any one of these storage devices. As used herein, the phrases "computer readable material" and "computer readable storage medium" refers to all computer-readable media, except for a transitory propagating signal *per se*.

Various embodiments and aspects of the disclosure will be described with reference to details discussed below. The following description and drawings are illustrative of the disclosure and are not to be construed as limiting the disclosure. Numerous specific details are described to provide a thorough understanding of various embodiments of the present disclosure. However, in certain instances, well-known or conventional details are not described in order to provide a concise discussion of embodiments of the present disclosure.

As used herein, the terms "comprises" and "comprising" are to be construed as being inclusive and open ended, and not exclusive. Specifically, when used in the specification and claims, the terms "comprises" and "comprising" and variations thereof mean the specified features, steps or components are included. These terms are not to be interpreted to exclude the presence of other features, steps or components.

As used herein, the term "exemplary" means "serving as an example, instance, or illustration," and should not be construed as preferred or advantageous over other configurations disclosed herein.

As used herein, the terms "about" and "approximately" are meant to cover variations that may exist in the upper and lower limits of the ranges of values, such as variations in properties, parameters, and dimensions. Unless otherwise specified, the terms "about" and "approximately" mean plus or minus 25 percent or less.

It is to be understood that unless otherwise specified, any specified range or group is as a shorthand way of referring to each and every member of a range or group individually, as well as each and every possible sub-range or sub-group encompassed therein and similarly with respect to any sub-ranges or sub-groups therein. Unless otherwise specified, the present disclosure relates to and explicitly incorporates each and every specific member and combination of sub-ranges or sub-groups.

As used herein, the term "on the order of", when used in conjunction with a quantity or parameter, refers to a range spanning approximately one tenth to ten times the stated quantity or parameter.

## Claims

1. A method of assessing a vibrometric response of a rigid structure via optical coherence tomography Doppler vibrometry comprising:

   employing an optical coherence tomography Doppler vibrometry system to interrogate the rigid structure along a depth axis, and obtaining an optical coherence tomography dataset, the optical coherence tomography dataset comprising an optical coherence tomography Doppler vibrometry dataset obtained while applying a stimulus;
   processing the optical coherence tomography dataset to identify a depth region, residing along the depth axis, that is associated with the rigid structure; and
   processing the optical coherence tomography Doppler vibrometry dataset to generate a vibrometric measure characterizing a vibrometric response of the rigid structure to the stimulus, wherein the vibrometric measure is generated based on non-uniformly weighted contributions from one or more subregions within the depth region associated with the rigid structure.

2. The method according to claim 1 wherein the non-uniformly weighted contributions are updated during a plurality of time intervals to provide a plurality of updated values of the non-uniformly weighted contributions, wherein each time interval is shorter in duration than a decoherence time of a speckle pattern, and wherein the updated values are employed to generate the vibrometric measure.

3. The method according to claim 1 or 2, wherein the non-uniformly weighted contribution from each subregion is dependent on a depth-domain A-line intensity measure associated with the subregion.

4. The method according to one of claims 1 to 3, wherein each subregion is sufficiently small to encompass speckle-induced spatially-dependent changes in depth-domain A-line intensity within the depth region.

5. The method according to claim 4, wherein each subregion corresponds to a depth-domain voxel.

6. The method according to one of claims 1 to 5, wherein the vibrometric measure is generated based on a contribution from a single subregion having a highest depth-domain A-line intensity measure among a set of subregions residing within the depth region.

7. The method according to one of claims 1 to 6, wherein the vibrometric measure is generated by:

   processing the optical coherence tomography Doppler vibrometry dataset to obtain a plurality of A-lines, and generating, from the plurality of A-lines, and for each subregion of a plurality of subregions residing with the depth region, an optical phase time series characterizing a temporal evolution of an optical phase during application of the stimulus, thereby obtaining a set of optical phase time series, each optical phase time series corresponding to a different subregion; and
   generating, from the set of optical phase time series, an aggregate optical phase time series, such that a given optical phase data point in the aggregate optical phase time series, corresponding to a given time point, is determined as a statistical measure characterizing a distribution of optical phase values among the plurality of subregions at the given time point; and

processing the aggregate optical phase time series to determine the vibrometric measure.

8. The method according to one of claims 1 to 7, wherein the optical coherence tomography Doppler vibrometry dataset is processed, prior to determining the vibrometric measure, to remove or reduce low-frequency motion noise.

9. The method according to one of claims 1 to 8, wherein the stimulus is an acoustic stimulus.

10. A system for assessing a vibrometric response of a rigid structure via optical coherence tomography Doppler vibrometry comprising:

an optical coherence tomography Doppler vibrometry system capable for applying a stimulus to the rigid structure; and
control and processing circuitry comprising at least one processor and associated memory, the memory comprising instructions executable by the at least one processor for performing operations comprising:

employing the optical coherence tomography Doppler vibrometry system to interrogate the rigid structure along a depth axis, and obtaining an optical coherence tomography dataset, the optical coherence tomography dataset comprising an optical coherence tomography Doppler vibrometry dataset obtained while applying the stimulus;
processing the optical coherence tomography dataset to identify a depth region, residing along the depth axis, that is associated with the rigid structure; and
processing the optical coherence tomography Doppler vibrometry dataset to generate a vibrometric measure characterizing a vibrometric response of the rigid structure to the stimulus, wherein the vibrometric measure is generated based on non-uniformly weighted contributions from one or more subregions within the depth region associated with the rigid structure.

11. The system according to claim 10, wherein the control and processing circuitry is configured such that the non-uniformly weighted contributions are updated during a plurality of time intervals to provide a plurality of updated values of the non-uniformly weighted contributions, wherein each time interval is shorter in duration than a decoherence time of a speckle pattern, and wherein the updated values are employed to generate the vibrometric measure.

12. The system according to claim 10 or 11, wherein the control and processing circuitry is configured such that the non-uniformly weighted contribution from each subregion is dependent on a depth-domain A-line intensity measure associated with the subregion.

13. The system according to one of claims 10 to 12, wherein the control and processing circuitry is configured such that each subregion is sufficiently small to encompass speckle-induced spatially-dependent changes in depth-domain A-line intensity within the depth region.

14. The system according to one of claims 10 to 13, wherein the control and processing circuitry is configured such that the vibrometric measure is generated based on a contribution from a single subregion having a highest depth-domain A-line intensity measure among a set of subregions residing within the depth region.

15. The system according to one of claims 10 to 14, wherein the control and processing circuitry is configured such that the vibrometric measure is generated by:

processing the optical coherence tomography Doppler vibrometry dataset to obtain a plurality of A-lines, and generating, from the plurality of A-lines, and for each subregion of a plurality of subregions residing with the depth region, an optical phase time series characterizing a temporal evolution of an optical phase during application of the stimulus, thereby obtaining a set of optical phase time series, each optical phase time series corresponding to a different subregion; and
generating, from the set of optical phase time series, an aggregate optical phase time series, such that a given optical phase data point in the aggregate optical phase time series, corresponding to a given time point, is determined as a statistical measure characterizing a distribution of optical phase values among the plurality of subregions at the given time point; and
processing the aggregate optical phase time series to determine the vibrometric measure.

100

118

126

128

120

122

122A

122

124B

SPK

DAC

DAC

DAC

112

124A

CONTROLLER
130

114

ADC
ATS9351

TIA

116

ADC

MIC

DAC

PUMP

122B

FIG. 1

**FIG. 2**

<u>300</u>

Employ Doppler OCT vibrometry system to interrogate rigid structure along a depth axis — 310

Obtain OCT dataset including Doppler OCT vibrometry dataset obtained during application of acoustic stimulus — 320

Process OCT dataset to identify depth region associated with rigid structure — 330

Process Doppler OCT vibrometry dataset to generate estimated vibration based on contributions from subregions within the depth region such that contribution from each subregion to the estimated vibration is dependent on depth domain intensity measure associated with subregion — 340

**FIG. 3**

**FIG. 4**

FIG. 5

EP 4 711 706 A1

Frequency-domain OCT dataset with sequence of interferograms H(k,z)
510

Time sequence of A-lines h(t,z)
512

Divide time sequence into M time intervals of duration T
514

Select depth
516

520

Calculate A-line magnitude |h(t,z)|
522

Estimate mean |h(t,z)|² over time
524

Identify voxels contained in rigid structure
526

Identify depth within rigid structure with maximum time-averaged |h(t,z)|²
528

For each time interval mT 530

Calculate signal energy sum(|h(t,z)|²) for the time interval
532

Estimate vibrometry measure x[z,m]
534

Calculate mean over time intervals
540

Estimate of vibrometry measure for rigid body
$\hat{x}$
540

FIG. 6

700

```
┌──────────────────┐     ┌──────────────────┐     ┌──────────────────┐     ┌─────────────────────────────────────────┐
│ Frequency-domain │     │ Time sequence of │     │                  │     │ For each time (each sample) 730         │
│ OCT dataset with │────▶│    A-lines       │────▶│ Calculate Φ<h(z,t)│────▶│  ┌──────────────────────────────────┐   │
│  sequence of     │     │    h(t,z)        │     │      714         │     │  │      Unwrap phase                │   │
│ interferograms   │     │     512          │     │                  │     │  │          732                     │   │
│   H(k,z)         │     │                  │     │                  │     │  └──────────────────────────────────┘   │
│    510           │     └──────────────────┘     └──────────────────┘     │                   │                      │
└──────────────────┘                                                       │                   ▼                      │
                                                                           │  ┌──────────────────────────────────┐   │
                                                                           │  │      Highpass filter             │   │
                                                                           │  │          734                     │   │
                                                                           │  └──────────────────────────────────┘   │
                                                                           │                   │                      │
  ┌─────────────────────────────────────────────────────────────────┐     │                   ▼                      │
  │ 520                                                              │     │  ┌──────────────────────────────────┐   │
  │ ┌────────────────┐  ┌────────────────┐  ┌────────────────┐       │     │  │ Calculate median of Φ            │   │
  │ │ Calculate A-line│  │ Estimate mean  │  │ Identify voxels│       │     │  │ across depth for                 │   │
  │ │  magnitude     │─▶│ |h(t,z)|² over  │─▶│ contained in   │──────────────▶│ voxels in the rigid              │   │
  │ │  |h(t,z)|      │  │  time          │  │ rigid          │       │     │  │ structure                        │   │
  │ │   522          │  │   524          │  │ structure      │       │     │  │  736                             │   │
  │ └────────────────┘  └────────────────┘  │   526          │       │     │  └──────────────────────────────────┘   │
  │                                         └────────────────┘       │     └─────────────────────────────────────────┘
  └─────────────────────────────────────────────────────────────────┘                         │
                                                                                               ▼
                                                                           ┌──────────────────────────────┐
                                                                           │ Calculate vibrometry         │
                                                                           │    measure                   │
                                                                           │      740                     │
                                                                           └──────────────────────────────┘
                                                                                               │
                                                                                               ▼
                                                                           ┌──────────────────────────────┐
                                                                           │     Estimate of              │
                                                                           │ vibrometry measure           │
                                                                           │ for rigid body x̂            │
                                                                           │      750                     │
                                                                           └──────────────────────────────┘
```

FIG. 7

EP 4 711 706 A1

## Simulated Histogram Results

**FIG. 8A**

| Method | Estimation | dB Error | Performance Relative to Best |
|---|---|---|---|
| Fitted Brightness Weighted | 3.946682e-13 | -248dB | 0 |
| Phase Median | 5.971600e-13 | -244dB | -4dB |
| Single Pixel FFT | 6.636754e-09 | -163dB | -185dB |
| Single Pixel Fit | 1.701914e-12 | -253dB | -13dB |

**FIG. 8B**

900

PROCESSOR
910

905

MEMORY
915

I/O DEVICES
920

COMMUNICATIONS
INTERFACE
925

EXTERNAL
STORAGE
930

DATA ACQUISITION
INTERFACE
935

OCT CONTROL MODULE
950

DFT PROCESSING
MODULE
970

FOCUS CHANGE
CONTROL MODULE
960

VIBROMETRY
PROCESSING MODULE
980

OCT SUBSYSTEM
990

HANDHELD OCT BEAM
DELIVERY AND IMAGING
DEVICE
995

FIG. 9

**EUROPEAN SEARCH REPORT**

Europäisches
Patentamt
European
Patent Office
Office européen
des brevets

**Application Number**

EP 24 20 0929

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2021/007605 A1 (ADAMSON ROBERT [CA] ET AL) 14 January 2021 (2021-01-14) * paragraphs [0062] - [0068], [0091], [0092]; figure 2 * | 1-15 | INV. G01B9/02 A61B5/00 G01B9/02091 |
| A | TAN ZHIWEI ET AL: "A Multi-Scale Fusion and Transformer Based Registration Guided Speckle Noise Reduction for OCT Images", IEEE TRANSACTIONS ON MEDICAL IMAGING, vol. 43, no. 1, 1 January 2024 (2024-01-01), pages 473-488, XP093239458, USA ISSN: 0278-0062, DOI: 10.1109/TMI.2023.3309813 * the whole document * | 1-15 | |
| A | MARK BREZINSKI: "Optical Coherence Tomography Principles and Applications", 31 December 2006 (2006-12-31), Elsevier B.V., XP040425666, ISBN: 978-0-12-133570-0 * chapter 11.2 * | 1-15 | |

TECHNICAL FIELDS
SEARCHED (IPC)

G01B
A61B

The present search report has been drawn up for all claims

| Place of search | Date of completion of the search | Examiner |
|---|---|---|
| Munich | 4 February 2025 | Biedermann, Benjamin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# EP 4 711 706 A1

## ANNEX TO THE EUROPEAN SEARCH REPORT
## ON EUROPEAN PATENT APPLICATION NO.

EP 24 20 0929

This annex lists the patent family members relating to the patent documents cited in the above-mentioned European search report.
The members are as contained in the European Patent Office EDP file on
The European Patent Office is in no way liable for these particulars which are merely given for the purpose of information.

04-02-2025

| Patent document cited in search report | Publication date | Patent family member(s) | | Publication date |
|---|---|---|---|---|
| US 2021007605 A1 | 14-01-2021 | EP | 3362787 A1 | 22-08-2018 |
| | | US | 2018256031 A1 | 13-09-2018 |
| | | US | 2021007605 A1 | 14-01-2021 |
| | | WO | 2017063090 A1 | 20-04-2017 |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CA 2018051255 W
- CA 2016051199 W
- CA 2018050200 W